# EUROPEAN PATENT SPECIFICATION

(11) **EP 2 838 559 B1**
(45) Date of publication and mention of the grant of the patent: **27.11.2019**
(21) Application number: 13718822.3
(22) Date of filing: 16.04.2013
(51) Int. Cl.: A61K 39/395, C07K 16/24, A61K 31/573

(54) **COMBINATION THERAPY OF ANTI-MIF ANTIBODIES AND GLUCOCORTICOIDS**
KOMBINATIONSTHERAPIE MIT ANTI-MIF-ANTIKÖRPERN UND GLUCOCORTICOIDEN
POLYTHÉRAPIE D'ANTICORPS ANTI-MIF ET DE GLUCOCORTICOÏDES

(30) Priority: 16.04.2012 US 201261624988 P; 06.07.2012 US 201261668864 P; 14.03.2013 US 201361785626 P
(43) Date of publication of application: 25.02.2015
(73) Proprietor: Baxalta GmbH, 6300 Zug (CH); Baxalta Incorporated, Bannockburn, IL 60015 (US)
(72) Inventor: KERSCHBAUMER, Randolf, A-3400 Klosterneuburg (AT); VOELKEL, Dirk, A-1220 Wien (AT); SCHEIFLINGER, Friedrich, A-1090 Wien (AT); EHRLICH, Hartmut, 75016 Paris (FR)
(74) Representative: Hoffmann Eitle
(86) International application number: PCT/EP2013/057892
(87) International publication number: WO 2013/156472

(56) References cited:
- WO-A1-94/26307
- WO-A2-01/64749
- WO-A2-03/060468
- CN-A- 1 869 207
- US-A1- 2003 099 653
- BAUGH & DONNELLY: "Macrophage migration inhibitory factor: a neuroendocrine modulator of chronic inflammation", JOURNAL OF ENDOCRINOLOGY, SOCIETY FOR ENDOCRINOLOGY, GB, vol. 179, no. 1, 1 October 2003 (2003-10-01), pages 15-23, XP002408028, ISSN: 0022-0795, DOI: 10.1677/JOE.0.1790015
- MAKITA H ET AL: "EFFECT OF ANTI-MACROPHAGE MIGRATION INHIBITORY FACTOR ANTIBODY ON LIPOPOLYSACCHARIDE-INDUCED PULMONARY NEUTROPHIL ACCUMULATION", AMERICAN JOURNAL OF RESPIRATORY AND CRITICAL CARE MEDICINE, AMERICAN LUNG ASSOCIATION, NEW YORK, NY, US, vol. 158, no. 2, 1 August 1998 (1998-08-01), pages 573-579, XP001120665, ISSN: 1073-449X
- SANTOS L ET AL: "ROLE OF MACROPHAGE MIGRATION INHIBITORY FACTOR (MIF) IN MURINE ANTIGEN-INDUCED ARTHRITIS: INTERACTION WITH GLUCOCORTICOIDS", CLINICAL AND EXPERIMENTAL IMMUNOLOGY, WILEY-BLACKWELL PUBLISHING LTD, GB, vol. 123, no. 2, 1 February 2001 (2001-02-01), pages 309-314, XP001122395, ISSN: 0009-9104, DOI: 10.1046/J.1365-2249.2001.01423.X
- KAR NENG LAI ET AL: "Role for macrophage migration inhibitory factor in acute respiratory distress syndrome", THE JOURNAL OF PATHOLOGY, vol. 199, no. 4, 1 April 2003 (2003-04-01) , pages 496-508, XP055068590, ISSN: 0022-3417, DOI: 10.1002/path.1291
- STOSIC-GRUJICIC S ET AL: "MIF in autoimmunity and novel therapeutic approaches", AUTOIMMUNITY REVIEWS, ELSEVIER, AMSTERDAM, NL, vol. 8, no. 3, 1 January 2009 (2009-01-01) , pages 244-249, XP025769813, ISSN: 1568-9972, DOI: 10.1016/J.AUTREV.2008.07.037 [retrieved on 2008-08-20]
- ISHIGURO Y ET AL: "Macrophage migration inhibitory factor has a proinflammatory activity via the p38 pathway in glucocorticoid-resistant ulcerative colitis", CLINICAL IMMUNOLOGY, ACADEMIC PRESS, US, vol. 120, no. 3, 1 September 2006 (2006-09-01), pages 335-341, XP024941335, ISSN: 1521-6616, DOI: 10.1016/J.CLIM.2006.05.010 [retrieved on 2006-09-01]
- AMANO T ET AL: "Blockade of macrophage migration inhibitory factor (MIF) prevents the antigen-induced response in a murine model of allergic airway inflammation", INFLAMMATION RESEARCH ; OFFICIAL JOURNAL OF: THE INTERNATIONAL ASSOCIATION OF INFLAMMATION SOCIETIES THE EUROPEAN HISTAMINE RESEARCH SOCIETY, BIRKHÄUSER-VERLAG, BA, vol. 56, no. 1, 1 January 2007 (2007-01-01), pages 24-31, XP019487517, ISSN: 1420-908X, DOI: 10.1007/S00011-007-5184-9
- SASHINAMI ET AL: "The role of macrophage migration inhibitory factor in lethal Listeria monocytogenes infection in mice", MICROBIAL PATHOGENESIS, ACADEMIC PRESS LIMITED, NEW YORK, NY, US, vol. 41, no. 2-3, 1 August 2006 (2006-08-01), pages 111-118, XP005598779, ISSN: 0882-4010, DOI: 10.1016/J.MICPATH.2006.06.001

## Description

The present invention pertains to anti-MIF antibodies, in particular their use in combination with glucocorticoids, in the treatment of MIF-related diseases.

### BACKGROUND

Macrophage migration inhibitory factor (MIF) is a cytokine initially isolated based upon its ability to inhibit the *in vitro* random migration of peritoneal exudate cells from tuberculin hypersensitive guinea pigs (containing macrophages) (Bloom et al. Science 1966, 153, 80-2; David et al. PNAS 1966, 56, 72-7). Today, MIF is known as a critical upstream regulator of the innate and acquired immune response that exerts a pleiotropic spectrum of activities.

The human MIF cDNA was cloned in 1989 (Weiser et al., PNAS 1989, 86, 7522-6), and its genomic localization was mapped to chromosome 22. The product of the human MIF gene is a protein with 114 amino acids (after cleavage of the N-terminal methionine) and an apparent molecular mass of about 12.5 kDa. MIF has no significant sequence homology to any other protein. The protein crystallizes as a trimer of identical subunits. Each monomer contains two antiparallel alpha-helices that pack against a four-stranded beta-sheet. The monomer has additional two beta-strands that interact with the beta-sheets of adjacent subunits to form the interface between monomers.

The three subunits are arranged to form a barrel containing a solvent-accessible channel that runs through the center of the protein along a molecular three-fold axis (Sun et al. PNAS 1996, 93, 5191-5196).

It was reported that MIF secretion from macrophages was induced at very low concentrations of glucocorticoids (Calandra et al. Nature 1995, 377, 68-71). However, MIF also counter-regulates the effects of glucocorticoids and stimulates the secretion of other cytokines such as tumor necrosis factor TNF-α and interleukin IL-1 β (Baugh et al., Crit Care Med 2002, 30, S27-35). MIF was also shown e.g. to exhibit pro-angiogenic, pro-proliferative and anti-apoptotic properties, thereby promoting tumor cell growth (Mitchell, R.A., Cellular Signalling, 2004. 16(1): p. 13-19; Lue, H. et al., Oncogene 2007.26(35): p. 5046-59). It is also e.g. directly associated with the growth of lymphoma, melanoma, and colon cancer (Nishihira et al. J Interferon Cytokine Res. 2000, 20:751-62).

MIF is a mediator of many pathologic conditions and thus associated with a variety of diseases including *inter alia* inflammatory bowel disease (IBD), rheumatoid arthritis (RA), acute respiratory distress syndrome (ARDS), asthma, glomerulonephritis, IgA nephropathy, myocardial infarction (MI), sepsis and cancer, though not limited thereto. Polyclonal and monoclonal anti-MIF antibodies have been developed against recombinant human MIF (Shimizu et al., FEBS Lett. 1996; 381, 199-202; Kawaguchi et al, Leukoc. Biol. 1986, 39, 223-232, and Weiser et al., Cell. Immunol. 1985, 90, 16778).

Anti-MIF antibodies have been suggested for therapeutic use. Calandra et al., (J. Inflamm. (1995); 47, 39-51) reportedly used anti-MIF antibodies to protect animals from experimentally induced gram-negative and gram-positive septic shock. Anti-MIF antibodies were suggested as a means of therapy to modulate cytokine production in septic shock and other inflammatory disease states.

US 6,645,493 discloses monoclonal anti-MIF antibodies derived from hybridoma cells, which neutralize the biological activity of MIF. It could be shown in an animal model that these mouse-derived anti-MIF antibodies had a beneficial effect in the treatment of endotoxin-induced shock.

US 200310235584 discloses methods of preparing high affinity antibodies to MIF in animals in which the MIF gene has been homozygously knocked-out.

Glycosylation-inhibiting factor (GIF) is a protein described by Galat et al. (Eur. J. Biochem, 1994, 224, 417-21). MIF and GIF are now recognized to be identical. Watarai et al. (PNAS 2000, 97, 13251-6) described polyclonal antibodies binding to different GIF epitopes to identify the biochemical nature of the posttranslational modification of GIF in Ts cells. Watarai et al, *supra,* reported that GIF occurs in different conformational isoforms *in vitro.* One type of isomer occurs by chemical modification of a single cysteine residue. The chemical modification leads to conformational changes within the GIF protein.

Glucocorticoids, sometimes also named glucocorticosteroids, are a class of steroid hormones that bind to the glucocorticoid receptor, which is present in almost every vertebrate animal. Glucocorticoids are part of the feedback mechanism of the immune system that turns down immune activity, i.e., inflammation. In medicine they are used to treat diseases that are caused by an overactive immune system, whereby exemplary for such diseases are allergies, asthma, autoimmune diseases and sepsis. They also interfere with some of the abnormal mechanisms in cancer cells, so that they are also used to treat cancer. Upon binding the glucocorticoid receptor, the activated glucocorticoid receptor complex up-regulates the expression of anti-inflammatory proteins in the nucleus by a process known as transactivation and represses the expression of pro-inflammatory proteins in the cytosol by attenuating actions on gene induction (via NF-κB, AP1, jun-jun-homodimers etc.). In principle, glucocorticoids are defined as a subgroup of corticosteroids. In addition, a novel class of compounds with glucocorticoid activity (SEGRA, selective glucocorticoid receptor agonists) are known. These compounds exert only some of the actions of full agonistic glucocorticoids, i.e., they fail to elicit the full spectrum of trans-activation, transrepression and indirect actions on gene induction (via NF-κB, AP1, jun-junhomdimers etc.). By analogy with selective estrogen receptor modulators (SERM's = tamoxifen, raloxifen,toremifen), these compounds are also referred to as selective glucocorticoid receptor modulators (SERM's). Examples of SEGRA's/SEGRM's include Mapracorate(= BOL303242X = ZK245186), compound A,RU24856, RU24782, RU40066, ZK 216348.

An important example of natural glucocorticoids is cortisol (or hydrocortisone) which is essential for life and regulates or supports a variety of important cardiovascular, metabolic, immunologic and homeostatic functions.

There are also several synthetic glucocorticoids available.

The glucocorticoids act in three major fields, i.e., the immunological, metabolic and foetal development field. In the immune field they upregulate the expression of anti-inflammatory proteins and downregulate the expression of proinflammatory proteins. Metabolic effects can be summarised as follows:
- Stimulation of gluconeogenesis, particularly in the liver: This pathway results in the synthesis of glucose from non-hexose substrates such as amino acids and glycerol from triglyceride breakdown and is particularly important in carnivores and certain herbivores. Enhancing the expression of enzymes involved in gluconeogenesis is probably the best-known metabolic function of glucocorticoids.
- Mobilization of amino acids from extrahepatic tissues: These serve as substrates for gluconeogenesis.
- Inhibition of glucose uptake in muscle and adipose tissue: The fatty acids released by lipolysis are used for production of energy in tissues like muscle, and the released glycerol provides another substrate for gluconeogenesis.

In foetal development glucocorticoids promote maturation of the lung and production of the surfactant which is necessary for extra-uterine lung function. They are further key substances for normal brain development.

A variety of synthetic glucocorticoids, some far more potent than cortisol, have been created for therapeutic use.

They differ in pharmacokinetics (absorption, half-life, volume of distribution, clearance) and in pharmacodynamics. Glucocorticoid potency, duration of effect, and overlapping mineralocorticoid potency varies. Cortisol (hydrocortisone) is the standard comparison for glucocorticoid potency. Hydrocortisone is the name used for pharmaceutical preparations of cortisol. Oral potency may be less than parenteral potency because significant amounts (up to 50% in some cases) may not be absorbed from the intestine.

Examples are:
Hydrocortisone, Cortisone acetate, cortisone/cortisol, Fluorocortolon, Prednisone, Prednisolone, Methylprednisolone,Triamcinolone, Dexamethasone, Betamethasone, Paramethasone.

In addition, there are compounds, which only mimic some but not all actions of glucocorticoids; these compounds are referred to as SEGRAs (selective glucocorticoid receptor agonists). Examples of SEGRA's/SEGRM's include Mapracorate(= BOL303242X = ZK245186), compound A,RU24856, RU24782, RU40066, ZK 216348.

Steroids mainly for local application (e.g. spray-lung, suppository-colon, cream-skin): Hydrocortisone, Beclomethasone, Budesonide, Fluticasone, Flunisolid, Mometasone, Ciclesonide, Clobetason.

It was very surprising to find that macrophage and T-cell MIF production was induced rather than inhibited by glucocorticoids in rodents (Calandra T, Bemhagen J, Metz CN et al., (1995) MIF as a glucocorticoid-induced modulator of cytokine production. Nature 377:68-71; and Bacher M, Metz CN, Calandra T, et al., (1996) An essential regulatory role for macrophage migration inhibitory factor in T-cell activation. PNAS 93: 7849-7854).

As MIF was known to have pro-inflammatory properties, these results appeared at first paradoxical and difficult to reconcile. Subsequently however it was found that MIF indeed overrides the anti-inflammatory immunosuppressive facts of glucocorticoids.

Although glucocorticoids have been shown to be useful and successful in the treatment of several different diseases and disorders (see below), the administration can have a range of side effects, depending on the type of medication used. Most common side effects include
- Immunosuppression
- Hyperglycemia due to increased gluconeogenesis, insulin resistance, and impaired glucose tolerance ("steroid diabetes")
- Increased skin fragility, easy bruising
- Negative calcium balance due to reduced intestinal calcium absorption
- Steroid-induced osteoporosis; reduced bone density (osteoporosis, osteonecrosis, higher fracture risk, slower fracture repair)
- Weight gain due to increased visceral and truncal fat deposition (central obesity) and appetite stimulation
- Adrenal insufficiency (if used for a long time and stopped suddenly without tapering off)
- Muscle breakdown (proteolysis), weakness; reduced muscle mass and repair
- Expansion of malar fat pads and dilation of small blood vessels in skin
- Anovulation, irregularity of menstrual periods
- Growth failure, pubertal delay
- Increased plasma amino acids, increased urea formation; negative nitrogen balance
- Excitatory effect on central nervous system (euphoria, psychosis)
- Glaucoma due to increased cranial pressure
- Cataracts

The combination of clinical problems produced by prolonged, excess glucocorticoids, whether synthetic or endogenous, is termed Cushing's syndrome.

In addition to the effects listed above, use of high dose steroids for more than a week begins to produce suppression of the patient's adrenal glands because the exogenous glucocorticoids suppress hypothalamic corticotropin-releasing hormone (CRH) and pituitary adrenocorticotropic hormone (ACTH). With prolonged suppression, the adrenal glands atrophy (physically shrink), and can take months to recover full function after discontinuation of the exogenous glucocorticoid.

During this recovery time, the patient is vulnerable to adrenal insufficiency during times of stress, such as illness.

Last but not least, patients, who received glucocorticoids over prolonged periods, can also develop glucocorticoid resistance. In these patients, even high doses of glucocorticoids do not suffice to elicit an adequate anti-inflammatory response (Barnes PJ, Adcock IM. Glucocorticoid resistance in inflammatory diseases. Lancet 2009 May 30; 373(9678):1905-1917).

The side effects listed above increase severely if the dosage of the given glucocorticoid drug is augmented. Decreasing the amount of the drug administered will usually also result in lower or alleviated side effects.

Thus, there remains an urgent need in the art for the provision of a therapy for MIF-related diseases and disorders which allows reduction of the administration dose of a given glucocorticoid.

### DESCRIPTION OF THE INVENTION

This object has been solved by the present invention.

In particular it could be shown that by a combination therapy of anti-MIF antibodies and a given glucocorticoid a synergistic effect could be detected which would allow treatment of MIF-related diseases with a lower dosage of the glucocorticoid and/or achieving a higher effect with a similar dosage as compared to treating this disease with the glucocorticoid alone.

In particular, a treatment by a combination therapy of anti-oxMIF antibodies and a given glucocorticoid could be shown to be associated with a synergistic effect, as described above and exemplified in the examples of the present invention.

Elevated MIF levels - i.e., levels of MIF in general - are detected after the onset of various diseases, *inter alia* after the onset of inflammatory diseases or cancer. However, MIF circulates also in healthy subjects, which makes a clear differentiation difficult. oxMIF, on the contrary, is not present in healthy subjects. oxMIF is increased in disease states and detectable in samples of patients, like e.g. blood, serum and urine.

It has been discovered after thorough research of MIF and antibodies thereto that the antibodies RAB9, RAB4 and RAB0 specifically bind to oxMIF (and are incapable of binding to redMiF). This binding is also observed for the antibodies RAM9, RAM4 and RAM0.

In earlier experiments carried out by the inventors, it could be shown that oxidative procedures like cystine-mediated oxidation, GSSG (ox. Glutathione)-mediated oxidation or incubation of MIF with Proclin300 or protein crosslinkers (e.g. BMOE) causes binding of MIF to the above-mentioned antibodies.

The surprising conclusions reached by the present inventors are:
- Redox modulation (Cystine/GSSG-mediated mild oxidation) of recombinant MIF (human, murine, rat, CHO, monkey)) or treatment of recombinant MIF with Proclin300 or protein crosslinkers leads to the binding of Baxter's anti-MIF antibodies RAB9, RAB4 and RAB0
- Reduction of oxMIF leads to the loss of Ab binding
- Specificity for oxMIF-isoforms correlates with biological Ab efficacy *in vivo.*
- oxMIF levels can be correlated with a disease state.

This additional knowledge regarding (ox)MIF served as a basis for the further studies of the present inventors.

Preferred embodiments of the present invention are:
1. An anti-MIF antibody in combination with a glucocorticoid for use in the treatment of a glucocorticoid-receptive disease.
2. The anti-MIF antibody in combination with a glucocorticoid according to item 1, where the glucocorticoid receptive disease is responsive to treatment with a glucocorticoid.
3. The anti-MIF antibody in combination with a glucocorticoid according to item 1 or 2 for use in the treatment of inflammation, allergy, cancer or asthma.
4. The anti-MIF antibody in combination with a glucocorticoid according to item 1, 2, or 3, wherein the antibody is an anti-oxMIF antibody.
5. The combination according to any one of items 1-4, wherein the anti-MIF antibody is selected from the following group: anti-MIF antibody RAB9, RAB4, RAB0, RAM9, RAM4 and/or RAM0.
6. The combination according to any of items 1 to 5 wherein the glucocorticoid is selected from the group consisting of glucocorticoid receptor agonists, e.g., Methylprednisolone, Prednisolone, Trimacinolone, Dexamethasone, Paramethasone, Fluorcortolone, Budesonide, Fluticasone, Flunisolide, Ciclesonide, Mometasone, Clobetasone, Cortisone and Hydrocortisone, and anti-inflammatory SEGRA's/SEGRM's (e.g., Mapracorate(= BOL303242X = ZK245186), compound A, RU24856, RU24782, RU4G066, ZK 216348).
7. The combination according to any of items 1 to 6, wherein the anti-MIF antibody is either one of antibodies RAB9, RAB4 or RAB0 or RAM9, RAM4 or RAM0, preferably antibody RAM9, the glucocorticoid is selected from the group of (methyl)Prednisolone and Dexamethasone in the case of systemic administration and Budesonide in the case of local administration and the MIF-related disease is inflammation, in particular nephritis, even more preferred lupus nephritis, gomerulonephritis, IgA or IgM nephropathy, systemic vasculitides (e.g., polyarteritis nodosa, Wegener's granulomatosis and Henoch-Schönlein purpura), anti-GBM nephritis and/or rapidly progressive glomerulonephritis, (type I, II, III or IV), like e.g. ANCA (anti-neutrophil cytoplasmic antibodies)nephritis).
8. The combination according to any of items 1 to 6, wherein the anti-MIF antibody is either one of antibodies RAB9, RAB4 or RAB0, or RAM9, RAM4 or RAM0 preferably antibody RAM9, the glucocorticoid is selected from the group of (methyl)Prednisolone and Dexamethasone in the case of systemic administration and Budesonide in the case of local administration and the MIF-related disease is Lupus nephritis.
9. The combination according to any of items 1 to 6, wherein the anti-MIF antibody is either one of antibodies RAB9, RAB4 or RAB0, or RAM9, RAM4 or RAM0, preferably RAM9, the glucocorticoid is selected from the group of (methyl)Prednisolone and Dexamethasone in the case of systemic administration and Budesonide in the case of local administration and the MIF-related disease is systemic lupus erythematosus.
10. The combination therapy according to any of items 1 to 6, wherein the anti-MIF antibody is either one of antibodies RAB9, RAB4 or RAB0, or RAM9, RAM4 or RAM0, preferably RAM9, the glucocorticoid is selected from the group of Dexamethasone, and (methyl)Prednisolone in the case of systemic administration and Budesonide in the case of local administration and the MIF-related disease is cutaneous inflammation.
11. The combination therapy according to any of items 1 to 6, wherein the anti-MIF antibody is either one of antibodies RAB9, RAB4 or RAB0, or RAM9, RAM4 or RAM0, preferably RAM9, the glucocorticoid is selected from the group of Dexamethasone, and (methyl)Prednisolone in the case of systemic administration and Budesonide or hydrocortisone in the case of local administration and the MIF-related disease is caused by a a T-cell mediated immune response.
12. The combination therapy of item 10 or 11, wherein the T-cell mediated immune response is cutaneous inflammation, e.g. psoriasis, or contact hypersensitivity.
13. The combination therapy of any one of items 10 -12, wherein the glucocorticoid is Budesonide and wherein the Budesonide is formulated for topical application.
14. The combination therapy as claimed in any of items 1 to 5, wherein the anti-MIF antibody is either one of antibodies RAB9, RAB4 or RAB0, or RAM9, RAM4 or RAM0, preferably RAM9, the glucocorticoid is Budesonide and the MIF-related disease is inflammatory bowel disease(IBD), wherein IBD is selected from ulcerative colitis and Crohn Disease.
15. The combination therapy as defined in any of items 1 to 6, wherein the anti-MIF antibody is either one of antibodies RAB9, RAB4 or RAB0, or RAM9, RAM4 or RAM0, preferably RAM9, the glucocorticoid is selected from the group of (methyl)Prednisolone and Dexamethasone in the case of systemic administration and Budesonide in the case of local administration and the MIF-related disease is multiple sclerosis.
16. A kit comprising the combination as defined in any of items 1 - 15 above, and instructions for use.
17. A combination as defined in any of items 1 - 15 above, or the kit of item 16, for use in the treatment of a glucocorticoid receptive disease, in particular inflammation, allergy, asthma or cancer.

The above mentioned antibodies are characterized and supported by both their sequences as well as by deposits as plasmids in *E*.*coli* (strain TG1), comprising either the light or the heavy chain of each of the above mentioned antibodies RAB0, RAB4 and RAB9, respectively and RAM0, RAM4 and RAM9, respectively.

The plasmids are characterized by their DSM number which is the official number as obtained upon deposit under the Budapest Treaty with the German Collection of Microorganisms, and Cell Cultures (DSMZ), Mascheroder Weg 1b, Braunschweig, Germany. The plasmids were deposited in *E. coli* strains, respectively.

The plasmid with the DSM 25110 number comprises the light chain sequence of the anti-MIF antibody RAB4.

The plasmid with the DSM 25112 number comprises the heavy chain (IgG4) sequence of the anti-MIF antibody RAB4.

The co-expression of plasmids DSM 25110 and DSM 25112 in a suitable host cell results in the production of preferred anti-MIF antibody RAB4.

The plasmid with the DSM 25111 number comprises the light chain sequence of the anti-MIF antibody RAB9.

The plasmid with the DSM 25113 number comprises the heavy chain (IgG4) sequence of the anti-MIF antibody RAB9.

The co-expression of plasmids DSM 25111 and DSM 25113 in a suitable host cell results in the production of preferred anti-MIF antibody RAB9.

The plasmid with the DSM 25114 number comprises the light chain sequence of the anti-MIF antibody RAB0.

The plasmid with the DSM 25115 number comprises the heavy chain (IgG4) sequence of the anti-MIF antibody RAB0.

The co-expression of plasmids DSM 25114 and DSM 25115 in a suitable host cell results in the production of preferred anti-MIF antibody RAB0.

Additional plasmids were deposited according to the Budapest Treaty on April 12, 2012 with the DSMZ for the light and heavy chain sequences of RAM0, RAM4 and RAM9, respectively, as following:
RAM9 (heavy chain): E.coli GA.662-01.pRAM9hc - DSM 25860.
RAM4 (light chain): E.coli GA.906-04.pRAM4lc - DSM 25861.
RAM9 (light chain): E.coli GA.661-01.pRAM9lc - DSM 25859.
RAM4 (heavy chain): E.coli GA.657-02.pRAM4hc - DSM 25862.
RAM0 (light chain): E.coli GA.906-01.pRAM0lc - DSM 25863.
RAM0 (heavy chain): E.coli GA.784-01.pRAM0hc - DSM 25864.

The term "prophylactic" or "therapeutic" treatment is art-recognized and refers to administration of a drug to a patient. If it is administered prior to clinical manifestation of the unwanted condition (e.g., disease or other unwanted state of the host animal) then the treatment is prophylactic, i.e., it protects the host against developing the unwanted condition, whereas if administered after manifestation of the unwanted condition, the treatment is therapeutic (i.e., it is intended to diminish, ameliorate or maintain the existing unwanted condition or side effects therefrom).

As used herein an anti-(ox)MIF compound refers to any agent that attenuates, inhibits, opposes, counteracts, or decreases the biological activity of (ox)MIF. An anti(ox)MIF compound may be an agent that inhibits or neutralizes (ox)MIF activity, for example an antibody, particularly preferred, the antibodies as described herein, even more preferred the antibodies RAB9, RAB4 and/or RAB0 or RAM9, RAM4 or RAM0.

The preferred MIF antagonist in accordance with the present invention is an anti-MIF antibody. Even more preferred the anti-MIF antibody is an antibody against oxMIF. In other embodiments, the anti-oxMIF antibodies, e.g. the antibodies mentioned above or an antigen-binding portion thereof bind oxMIF with a K_{D} of less than 100 nM, preferably a K_{D} of less than 50 nM, even more preferred with a K_{D} of less than 10nM.

The invention further relates to kits comprising an anti-MIF antibody or an antigen-binding portion thereof as well as a glucocorticoid agent according to the invention. A kit may include in addition to the antibody and the glucocorticoid agent, further therapeutic agents and uses thereof. A kit also can include instructions for use in a therapeutic method.

Earlier results have shown that an anti-MIF antibody that only binds oxMIF and does not bind redMIF and further inhibits GCO and/or cell proliferation induces a beneficial effect in an animal model.

### DETAILED DESCRIPTION OF THE INVENTION

The invention is further described in the figures as enclosed.

### Description of the figures:

- **Figure 1:**: Schematic diagram of dose-finding treatment with Dexamethasone as carried out in Example 1a.
- **Figure 2:**: Proteinuria on day 8 after administration of several different doses of Dexamethasone.
- **Figure 3:**: Macrophage infiltration after administration of several doses of Dexamethasone. The number of macrophages (ED1 positive cells) per glomerular cross section was determined. The result for each animal as well as the mean for each group is shown.
- **Figure 4:**: Glomerular crescents after several doses of Dexamethasone. The percentage of glomerular crescents determined for each animal is indicated and the mean for each group is shown.
- **Figure 5:**: Schematic diagram of the dose finding for anti-MIF antibody RAM9 in a glycine formulation according to Example 1b.
- **Figure 6:**: Proteinuria on day 8 after two doses of antibody RAM9.
- **Figure 7:**: Macrophage infiltration after two doses of antibody RAM9. The number of macrophages (ED1 positive cells) per glomerular cross section was determined. The result for each animal as well as the mean for each group is shown
- **Figure 8:**: Glomerular crescent formation after two doses of antibody RAM9. The percentage of glomerular crescents determined for each animal is indicated and the mean for each group is shown.
- **Figure 9:**: Schematic diagram of treatment schedule for a combination of Dexamethasone and antibody RAM9 according to Example 1c.
- **Figure 10:**: Reduction of proteinuria after combined treatment with antibody RAM9 (dose range 0-120 mg/kg) and Dexamethasone (0.025 mg/kg). The mean of two independent experiments is depicted. The arrows show the % reduction of proteinuria by different doses of Dexamethasone without application of antibody.
- **Figure 11:**: Observed reduction of macrophage infiltration after combined treatment with antibody RAM9 (dose range 0-120 mg/kg) and Dexamethasone (0.025 mg/kg). The mean of two independent experiments is depicted. The arrows show the % reduction of macrophage infiltration by different doses of Dexamethasone without application of antibody.
- **Figure 12:**: Reduction of crescent formation after combined treatment with antibody RAM9 (dose range 0-120 mg/kg) and Dexamethasone (0.025 mg/kg). The mean of two independent experiments is depicted. The arrows show the % reduction of crescent formation by the different doses of Dexamethasone without application of antibody.
- **Figure 13:**: *Contact hypersensitivity (CHS) response to DNFB.* Attenuated CHS reaction on the challenged ear of mice treated with anti-MIF (RAM9) in combination with hydrocortisone (+HC) compared with mice treated with RAM9 without application of hydrocortisone (-HC). Isotype control antibody treated mice (with or without topical application of hydrocortisone) were used as negative control. Eight mice were used per group and reduced ear contact dermatitis was quantified by measuring the difference of ear thickness between the right (challenged) and the left (non-challenged) ear.

### Definitions and General Techniques

Unless otherwise defined herein, scientific and technical terms used in connection with the present invention shall have the meanings that are commonly understood by those of ordinary skill in the art. Generally, nomenclatures used in connection with, and techniques of, cell and tissue culture, molecular biology, immunology, microbiology, genetics and protein and nucleic acid chemistry described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2nd ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990), which are incorporated herein by reference.

"MIF" or "macrophage migration inhibitory factor" refers to the protein, which is known as a critical mediator in the immune and inflammatory response, and as a counterregulator of glucocorticoids. MIF includes mammalian MIF, specifically human MIF (Swiss-Prot primary accession number: P14174), wherein the monomeric form is encoded as a 115 amino acid protein but is produced as a 114 amino acid protein due to cleavage of the initial methionine. "MIF" also includes "GIF" (glycosylation-inhibiting factor) and other forms of MIF such as fusion proteins of MIF.

The numbering of the amino acids of MIF starts with the N-terminal methionine (amino acid 1) and ends with the C-terminal alanine (amino acid 115).

"oxidized MIF" or oxMIF is defined for the purposes of the invention as an isoform of MIF that occurs by treatment of MIF with mild oxidizing reagents, such as Cystine. As has been shown by the present invention, recombinant oxMIF that has been treated this way comprises isoform(s) of MIF that share structural rearrangements with oxMIF that (e.g.) occurs *in vivo* after challenge of animals with bacteria.

redMIF is defined for the purposes of this invention as reduced MIF and is MIF which does not bind to RAB0, RAB9 and/or RAB4.

The anti-oxMIF antibodies described in this invention are able to discriminate between ox and red MIF, which are generated by mild oxidation or reduction, respectively. The anti-oxMIF antibodies are useful to specifically detect oxMIF. Discrimination between these conformers is assessed by ELISA or surface plasmon resonance. Both techniques can be performed as well known to a person skilled in the art and as described below.

### Assessing differential binding of the antibodies by Biacore.

Binding kinetics of oxMIF and redMIF to antibody RAB9 and RAB0 are examined by surface plasmon resonance analysis using a Biacore 3000 System. The antibodies were coated on a CM5 (= carboxymethylated dextran) chip and recombinant MIF protein, pre-incubated with 0.2% Proclin300, were injected. (Proclin300 consists of oxidative isothiazolones that stabilize the oxMIF structure). In native HBS-EP buffer (= Biacore running buffer) without addition of ProClin300, none of the recombinant MIF proteins bound to RAB9, RAB0 or to the reference antibody (irrelevant isotype control antibody) used as negative (background) binding control.

In a preferred embodiment, oxMIF is MIF which is differentially bound by antibody RAB9, RAB4 and/or RAB0 or an antigen-binding fragment thereof, meaning that these antibodies do bind to oxMIF while redMIF is not bound by either one of these antibodies.

In other embodiments, the anti-oxMIF antibodies, e.g., the antibodies mentioned above or an antigen-binding portion thereof bind oxMIF with a K_{D} of less than 100 nM, preferably a K_{D} of less than 50 nM, even more preferred with a K_{D} of less than 10nM, In an even more preferred embodiment, the antibodies bind oxMIF with a K_{D} of less than 5 nM.

(Non-)binding of an antibody, e.g. RAB9, RAB4 or RAB0 (to oxMIF or redMIF) can be determined as generally known to a person skilled in the art, examples being any one of the following methods: ELISA with recombinant MIF in its reduced or oxidized state, or surface plasmon resonance using recombinant MIF in its reduced or oxidized state, like the well known Biacore assay, described above.

A preferred method for the determination of binding is surface plasmon resonance of an antibody to e.g. rec. (ox)MIF whereupon "binding" is meant to be represented by a K_{D} of less than 100 nM preferably less than 50 nM, even more preferred less than 10 nM whereas the non-binding to redMIF is characterized by a K_{D} of more than 400 nM. "Binding" and "specific binding" is used interchangeably here to denote the above. "Differential binding" in the context of this application means that a compound, in particular the antibodies as described herein, bind to oxMIF (e.g., with the K_{D} values mentioned above) while they do not bind to redMIF (with non-binding again being defined as above).

An "antibody" refers to an intact antibody or an antigen-binding portion that competes with the intact antibody for (specific) binding. See generally, Fundamental Immunology, Ch. 7 (Paul, W., ed., 2nd ed. Raven Press, N.Y. (1989)) (incorporated by reference). The term antibody includes human antibodies, mammalian antibodies, isolated antibodies and genetically engineered forms such as chimeric, camelized or humanized antibodies, though not being limited thereto.

The term "antigen-binding portion" of an antibody refers to one or more fragments of an antibody that retain the ability to specifically bind to an antigen (e.g.(ox)MIF). Antigen-binding portions may be produced by recombinant DNA techniques or by enzymatic or chemical cleavage of intact antibodies. Antigen-binding portions include e.g. - though not limited thereto -the following: Fab, Fab', F(ab')2, Fv, and complementarity determining region (CDR) fragments, single-chain antibodies (scFv), chimeric antibodies, antibodies and polypeptides that contain at least a portion of an antibody that is sufficient to confer specific antigen binding to the polypeptide, i.e., ox or redMIF. From N-terminus to C-terminus, both the mature light and heavy chain variable domains comprise the regions FR1, CDR1, FR2, CDR2, FR3, CDR3 and FR4. The assignment of amino acids to each domain is in accordance with the definitions of Kabat, Sequences of Proteins of Immunological Interest (National Institutes of Health, Bethesda, Md. (1987 and 1991)), Chothia et al. J. Mol. Biol. 196:901-917 (1987), or Chothia et al., Nature 342:878-883 (1989). An antibody or antigen-binding portion thereof can be derivatized or linked to another functional molecule (e.g., another peptide or protein). For example, an antibody or antigen- binding portion thereof can be functionally linked to one or more other molecular entities, such as another antibody (e.g., a bispecific antibody or a diabody), a detectable agent, a cytotoxic agent, a pharmaceutical agent, and/or a linking molecule.

The term "K_{D}" refers here, in accordance with the general knowledge of a person skilled in the art to the equilibrium dissociation constant of a particular antibody with the respective antigen. This equilibrium dissociation constant measures the affinity. The affinity determines how much complex is formed at equilibrium (steady state where association balances dissociation) (here: ox or redMIF and antibody).
kₐ= association rate constant[M⁻¹ s⁻¹]
k_{d} =dissociation rate constant[s⁻¹]
K_{D} =equilibrium dissociation constant = kd/ka [M]

The term "human antibody" refers to any antibody in which the variable and constant domains are human sequences. The term encompasses antibodies with sequences derived from human genes, but which have been changed, e.g. to decrease possible immunogenicity, increase affinity, eliminate cysteines that might cause undesirable folding, etc. The term encompasses such antibodies produced recombinantly in non-human cells, which might e.g. impart glycosylation not typical of human cells.

The term "humanized antibody" refers to antibodies comprising human sequences and containing also non-human sequences; in particular, a "humanized antibody" refers to a non-human antibody where human sequences have been added and/or replace the non-human sequences.

The term "camelized antibody" refers to antibodies wherein the antibody structure or sequences has been changed to more closely resemble antibodies from camels, also designated camelid antibodies. Methods for the design and production of camelized antibodies are part of the general knowledge of a person skilled in the art.

The term "chimeric antibody" refers to an antibody that comprises regions from two or more different species.

The term "isolated antibody" or "isolated antigen-binding portion thereof" refers to an antibody or an antigen-binding portion thereof that has been identified and selected from an antibody source such as a phage display library or a B-cell repertoire.

The production of the anti-(ox)MIF antibodies according to the present invention includes any method for the generation of recombinant DNA by genetic engineering, e.g. via reverse transcription of RNA and/or amplification of DNA and cloning into expression vectors. In some embodiments, the vector is a viral vector, wherein additional DNA segments may be ligated into the viral genome. In some embodiments, the vector is capable of autonomous replication in a host cell into which it is introduced (e.g., bacterial vectors having a bacterial origin of replication and episomal mammalian vectors). In other embodiments, the vector (e.g. non-episomal mammalian vectors) can be integrated into the genome of a host cell upon introduction into the host cell, and thereby replicated along with the host genome. Moreover, certain vectors are capable of directing the expression of genes to which they are operatively linked. Such vectors are referred to herein as "recombinant expression vectors" (or simply, "expression vectors").

Anti-(ox)MIF antibodies can be produced *inter alia* by means of conventional expression vectors, such as bacterial vectors (e.g., pBR322 and its derivatives), or eukaryotic vectors. Those sequences that encode the antibody can be provided with regulatory sequences that regulate the replication, expression and/or secretion from the host cell.

These regulatory sequences comprise, for instance, promoters (e.g., CMV or SV40) and signal sequences. The expression vectors can also comprise selection and amplification markers, such as the dihydrofolate reductase gene (DHFR), hygromycin-B-phosphotransferase, and thymidine-kinase. The components of the vectors used, such as selection markers, replicons, enhancers, can either be commercially obtained or prepared by means of conventional methods. The vectors can be constructed for the expression in various cell cultures, e.g., in mammalian cells such as CHO, COS, HEK293, NSO, fibroblasts, insect cells, yeast or bacteria such as *E*.*coli*. In some instances, cells are used that allow for optimal glycosylation of the expressed protein.

The anti-(ox)MIF antibody light chain gene(s) and the anti-(ox)MIF antibody heavy chain gene(s) can be inserted into separate vectors or the genes are inserted into the same expression vector. The antibody genes are inserted into the expression vector by standard methods, e.g., ligation of complementary restriction sites on the antibody gene fragment and vector, or blunt end ligation if no restriction sites are present.

The production of anti-(ox)MIF antibodies or antigen-binding fragments thereof may include any method known in the art for the introduction of recombinant DNA into eukaryotic cells by transfection, e.g. via electroporation or microinjection. For example, the recombinant expression of anti-(ox)MIF antibody can be achieved by introducing an expression plasmid containing the anti-(ox)MIF antibody encoding DNA sequence under the control of one or more regulating sequences such as a strong promoter, into a suitable host cell line, by an appropriate transfection method resulting in cells having the introduced sequences stably integrated into the genome. The lipofection method is an example of a transfection method, which may be used according to the present invention.

The production of anti-(ox)MIF antibodies may also include any method known in the art for the cultivation of said transformed cells, e.g., in a continuous or batchwise manner, and the expression of the anti-(ox)MIF antibody, e.g., constitutive or upon induction. It is referred in particular to WO 2009/086920 for further reference for the production of anti-(ox)MIF antibodies. In a preferred embodiment, the anti-(ox)MIF antibodies as produced according to the present invention bind to oxMIF or an epitope thereof. Particularly preferred antibodies in accordance with the present invention are antibodies RAB9, RAB4 and/or RAB0. Alternative preferred antibodies are RAM9, RAM 4 and/or RAM0.

The sequences of these antibodies are disclosed in WO 2009/086920; see in addition the sequence list of the present application and the following:

The anti-MIF antibody of the invention is preferably an isolated monoclonal antibody. The anti-MIF antibody can be an IgG, an IgM, an IgE, an IgA, or an IgD molecule. In other embodiments, the anti-MIF antibody is an IgG1, IgG2, IgG3 or IgG4 subclass. In other embodiments, the antibody is either subclass IgG1 or IgG4. In other embodiments, the antibody is subclass IgG4. In some embodiments, the IgG4 antibody has a single mutation changing the serine (serine 228) to proline. Accordingly, the CPSC sub-sequence in the Fc region of IgG4 becomes CPPC, which is a sub-sequence in IgG1 (Angal et al. MolImmunol. 1993, 30, 105-108).

Additionally, the production of anti-(ox)MIF antibodies may include any method known in the art for the purification of an antibody, e.g., via anion exchange chromatography or affinity chromatography. In one embodiment the anti-(ox)MIF antibody can be purified by size exclusion chromatography.

The terms "center region" and "C-terminal region" of MIF refer to the region of human MIF comprising amino acids 35-68 and aa 86-115, respectively, preferably aa 50-68 and aa 86 to 102 of human MIF, respectively.

Particularly preferred antibodies of the present invention bind to either region aa 50-68 or region aa 86-102 of human MIF. This is also reflected by the epitope binding of the preferred antibodies RAB0, RAB4 RAB2 and RAB9 as well as RAM4, RAM0 and RAM9 which bind as follows:

| | |
|---|---|
| RAB4 and RAM4: | aa 86-102 |
| RAB9 and RAM9: | aa 50-68 |
| RAB0 and RAM0: | aa 86-102 |
| RAB2: | aa 86 - 102 |

Thus, RAM4 and RAB4 have the same specificity, as have RAM9 and RAB9. The same is true for RAM0 and RAB0. This is also reflected by the examples carried out which show that similar results will be obtained with these antibodies.

The term "epitope" includes any protein determinant capable of specific binding to an immunoglobulin or an antibody fragment. Epitopic determinants usually consist of chemically active surface groupings of molecules such as exposed amino acids, amino sugars, or other carbohydrate side chains and usually have specific three-dimensional structural characteristics, as well as specific charge characteristics.

The term "vector" refers to a nucleic acid molecule capable of transporting another nucleic acid to which it has been linked. In some embodiments, the vector is a plasmid, i.e., a circular double stranded DNA loop into which additional DNA segments may be ligated.

The term "host cell" refers to a cell line, which is capable to produce a recombinant protein after introducing an expression vector. The term "recombinant cell line", refers to a cell line into which a recombinant expression vector has been introduced. It should be understood that "recombinant cell line" means not only the particular subject cell line but also the progeny of such a cell line. Because certain modifications may occur in succeeding generations due to either mutation or environmental influences, such progeny may not, in fact, be identical to the parent cell, but are still included within the scope of the term "recombinant cell line" as used herein.

The host cell type according to the present invention is e.g. a COS cell, a CHO cell or e.g. an HEK293 cell, or any other host cell known to a person skilled in the art, thus also for example including bacterial cells, like e.g. *E.coli* cells. In one embodiment, the anti-MIF antibody is expressed in a DHFR-deficient CHO cell line, e.g., DXB11, and with the addition of G418 as a selection marker. When recombinant expression vectors encoding antibody genes are introduced into CHO host cells, the antibodies are produced by culturing the host cells for a period of time sufficient to allow for expression of the antibody in the host cells or secretion of the antibody into the culture medium in which the host cells are grown.

Anti-(ox)MIF antibodies can be recovered from the culture medium using standard protein purification methods.

The second active ingredient of the combination therapy as provided by the present invention is a glucocorticoid. Glucocorticoids, sometimes also named glucocorticosteroids, are a class of steroid hormones that bind to the glucocorticoid receptor (Glucocorticoid receptor a), which is present in almost every vertebrate animal.

Glucocorticoids are part of the feedback mechanism of the immune system that turns immune activity, i.e. inflammation, down. In medicine they are used to treat diseases that are caused by an overactive immune system, whereby exemplary for such diseases are allergies, asthma, autoimmune diseases and sepsis. They also interfere with some of the abnormal mechanisms in cancer cells, so that they are also used to treat cancer. Upon binding the glucocorticoid receptor, the activated glucocorticoid receptor complex up-regulates the expression of anti-inflammatory proteins in the nucleus by a process known as transactivation and represses the expression of pro-inflammatory proteins in the cytosol. In principle, glucocorticoids are defined as a subgroup of cortico-steroids. In addition, a novel class of compounds with glucocorticoid activity (SEGRA, selective glucocorticoid receptor agonists) are known.

These compounds exert only some of the actions of full agonistic glucocorticoids, i.e., they fail to elicit the full spectrum of transactivation, transrepression and indirect actions on gene induction (via NF-κB, AP1, jun-jun homodimers etc.). By analogy with selective estrogen receptor modulators (SERM's = tamoxifen, raloxifen, toremifen), these compounds are also referred to as selective glucocorticoid receptor modulators (SEGRM's). Examples of SEGRA's/SEGRM's include Mapracorate(= BOL303242X = ZK245186), compound A,RU24856, RU24782, RU40066, ZK 216348. All these compounds have been described in the art and are by now well known to the person skilled in the art. See in particular review article De Bosscher et al, 2010, Curr Opin Pharmacol 10: 497-504 for an overview. RU 24856, RU 40066 and RU 24782 are described e.g. in Vayssiere et al, 1997, Mol Endocrinol 11:1245-1255. These are the respective chemical formulae:

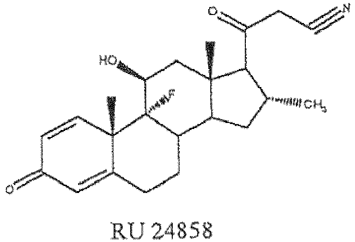

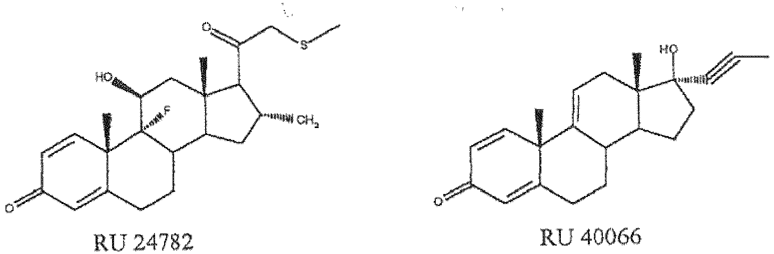

ZK245186 is disclosed in Proksch et al, 2011, Drug Metab Dispos 39:1181-1187 and in Schäcke et al., 2009, Br J Pharmacol, 158(4):1088-103. It has the following formula: (R)-1,1,1-Trifluoro-4-(5-fluoro-2,3-dihydrobenzofuran-7-yl)-4-methyl-2-{[(2-methyl-5-quinolyl)amino]methyl}pentan-2-ol. ZK 216348 is known from Schäcke et al.,2004, PNAS USA 101:227-232. It has the following chemical formula:

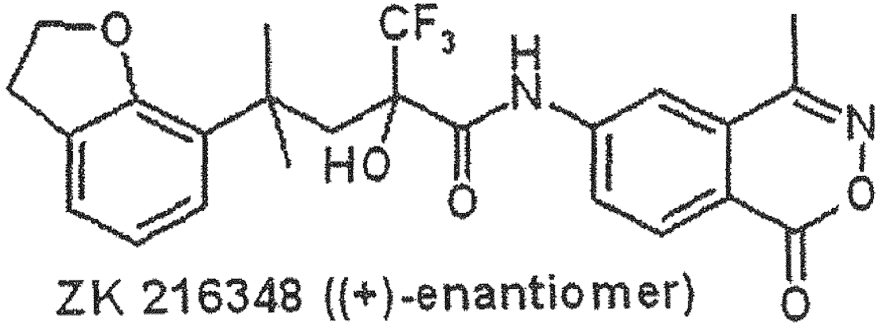

Compound A is disclosed in Rauner et al, 2011, Endocrinology 152:103-112 as well as in De Bosscher K, Vanden Berghe W, Beck IM, Van Molle W, Hennuyer N, Hapgood J, Libert C, Staels B, Louw A, Haegeman G, 2005, A fully dissociated compound of plant origin for inflammatory gene repression. Proc Natl Acad Sci USA 102:15827-15832. The Compound A has the formula 2-(4-acetoxyphenyl)-2-chloro-N-methyl-ethylammonium chloride.

An important example of a glucocorticoid is cortisol (or hydrocortisone), which is essential for life and regulates or supports a variety of important cardiovascular, metabolic, immunologic and homeostatic functions. There are also several synthetic glucocorticoids available.

The glucocorticoids act in three major fields, i.e. the immunological, metabolic and foetal development field. In the immune field they upregulate the expression of anti-inflammatory proteins and downregulate the expression of proinflammatory proteins. Metabolic effects can be summarised as follows:
- Stimulation of gluconeogenesis, particularly in the liver: This pathway results in the synthesis of glucose from non-hexose substrates such as amino acids and glycerol from triglyceride breakdown and is particularly important in carnivores and certain herbivores. Enhancing the expression of enzymes involved in gluconeogenesis is probably the best-known metabolic function of glucocorticoids.
- Mobilization of amino acids from extrahepatic tissues: These serve as substrates for gluconeogenesis.
- Inhibition of glucose uptake in muscle and adipose tissue: The fatty acids released by lipolysis are used for production of energy in tissues like muscle, and the released glycerol provides another substrate for gluconeogenesis.

In foetal development glucocorticoids promote maturation of the lung and production of the surfactant which is necessary for extra-uterine lung function. They are further key substances for normal brain development.

A variety of synthetic glucocorticoids, some far more potent than cortisol, have been created for therapeutic use.

They differ in pharmacokinetics (absorption factor, half-life, volume of distribution, clearance) and in pharmacodynamics.

In the context of the present invention "glucocorticoids" shall mean all glucocorticoids which act at the glucocorticoids receptor alpha. In particular, this encompasses the "classical" glucocorticoid receptor agonists as well as the above described SEGRAs. In a preferred embodiment, all those glucocorticoids are encompassed which act via the NF-κB pathway and/or the AP-1 pathway. In a further preferred embodiment, the inventive glucocorticoids are all ("classical") glucocorticoids.

Theoretically, at some point in time in the future there might be - though unlikely - glucocorticoids which only act e.g. via transactivation or transrepression, but do not act via the NF-κB pathway. In a preferred embodiment, these compounds would not be encompassed by the present invention.

NF-κB is nuclear factor kappa light chain enhancer of activated B-cells. It is a specific transcription factor, which exists in practically all cell types and tissues. By binding to particular regulatory regions of the DNA it can influence the transcription of target genes, like anti-apoptotic genes, the caspase-family of genes, and in particular cyclooxygenase-2 (Cox-2), IL-6 and TNF-alpha.

Glucocorticoid potency, duration of effect, and overlapping mineralocorticoid potency varies. Cortisol (hydrocortisone) is the standard comparison for glucocorticoid potency. Hydrocortisone is the name used for pharmaceutical preparations of cortisol. Oral potency may be less than parenteral potency because significant amounts (up to 50% in some cases) may not be absorbed from the intestine.

Examples of preferred glucocorticoids of the present invention are:
Hydrocortisone, Cortisone acetate, Cortisone/Cortisol, Fluorocortolon,
Prednisone,Prednisolone,Methylprednisolone (particularly for systemic administration), Triamcinolone,
Dexamethasone, Betamethasone, Paramethasone, Budesonide (particularly for local administration), SEGRAs (selective glucocorticoid receptor agonists).

In addition, there are compounds, which only mimic some but not all actions of glucocorticoids; these compounds are referred to as SEGRAs (selective glucocorticoid receptor agonists). Examples of SEGRA's/SEGRM's include Mapracorate(= BOL303242X = ZK245186), compound A, RU24856, RU24782, RU40066, ZK 216348. SEGRAs typically exert their effects via the NFκ-B pathway.

Steroids mainly for local application (e.g. spray-lung, suppository-colon, cream-skin): Hydrocortisone, Beclomethasone, Budesonide, Fluticasone, Flunisolid, Mometasone, Ciclesonide, Clobetason, most preferably Budesonide.

In a further preferred embodiment the glucocorticoid is selected from the group consisting of Hydrocortisone, Cortisone acetate, Budesonide, Fludrocortisone, Dexamethasone, Prednisolone and/or Methylprednisolone.

Particularly preferred are (Methyl)prednisolon, Hydrocortisone and Dexamethasone, even more preferred (methyl)Prednisolone for systemic administration. Hydrocortisone can be preferred for a topical application in one embodiment of the invention. In a further preferred embodiment Budesonide is preferred for local administration.

Salts, esters and/or isomers of glucocorticoids are all meant to be encompassed in the scope of the present invention and shall be understood to fall under the term "glucocorticoid". They are all well known or can be prepared by well-known methods.

It was very surprising to find that macrophage and T-cell MIF production was induced rather than inhibited by glucocorticoids in rodents (Calandra T, Bernhagen J, Metz CN et al.,(1995) MIF as a glucocorticoid-induced modulator of cytokine production. Nature 377:68-71; and Bacher M, Metz CN, Calandra T, et al., (1996) An essential regulatory role for macrophage migration inhibitory factor in T-cell activation. PNAS 93: 7849-7854).

As MIF was known to have pro-inflammatory properties, these results appeared at first paradoxical and difficult to reconcile. Subsequently however it was found that MIF indeed overrides the anti-inflammatory immunosuppressive facts of glucocorticoids.

Any given glucocorticoid known by a person skilled in the art can be used in the context of the present invention; these can be the above-mentioned exemplary glucocorticoids. Further, the synthesis of a glucocorticoid again is well-known in the art.

Glucocorticoids have been shown to be successful in alleviation and treatment of several diseases. However, most glucocorticoids are associated with a range of side effects, as e.g. described above, which are in some cases extreme, to the extent that the treatment has to be abrogated. In any case, the side effects place a further burden on the physical and mental health of a patient and should thus be avoided as far as possible.

With the present invention, by combining a glucocorticoid with an anti-MIF antibody it is now possible to reduce the amount of the glucocorticoid, which is necessary for a given treatment compared to a situation where the glucocorticoid is given as the sole active ingredient. A further possibility enabled by the present invention is to maintain the dose of the glucocorticoid as compared to the glucocorticoid given alone and have a much higher treatment response in the patient.

It was shown quite surprisingly by the present inventors that the effect obtained by combining a glucocorticoid with an anti-MIF antibody allowed a clear dose reduction of glucocorticoid while the same treatment response could still be maintained.

A treatment response can easily be determined by a person skilled in the art and refers to diminishing or ameliorating or alleviating a given condition. Assays to determine this are well known and can be for example determination of the likelihood or length of survival of a subject having a disease with the likelihood or length of survival in other subjects having the same disease or by determining the change of symptoms within one and the same patient over a period of time.

A suitable assay is for example the erythrocyte sedimentation assay; additional assays are those as described in the present examples. Preferred glucocorticoids according to the present invention are Cortisone-acetate, Hydrocortisone, Fludrocortisone, Dexamethasone, Budesonide, Prednisolone and/or Methylprednisolone.

Particularly preferred are Methylprednisolon, Hydrocortisone and Budesonide and Dexamethasone. Even more preferred are (methyl)Prednisolone and Dexamethasone for systemic administration and Budesonide for topical administration.

Particularly preferred combinations are
- RAM9 in combination with Dexamethasone,
- RAM9 in combination with Budesonide,
- RAM9 in combination with hydrocortisone,
- RAM9 in combination with Prednisolone or Methylprednisolone,
- RAM0 in combination with Dexamethasone,
- RAM4 in combination with Dexamethasone,
- RAM0 in combination with Budesonide,
- RAM4 in combination with Budesonide,
- RAM0 in combination with Prednisolone or Methylprednisolone,
- RAM4 in combination with Prednisolone or Methylprednisolone,
- RAM4 in combination with hydrocortisone,
- RAM0 in combination with hydrocortisone.

In a particular preferred embodiment Hydrocortisone and Budesonide is used for topical/local application.

It is explicitly pointed out that in the context of the present invention every suitable glucocorticoid is encompassed which is known for the treatment of a particular disease state. Thus, in the most preferred embodiment, the glucocorticoid of the invention is a glucocorticoid which is the respective gold standard treatment for a given disease.

The above preferred combinations are used preferably for the treatment of a glucocorticoid receptive disease as defined hereinafter.

The present combination therapy is particularly suitable for the treatment of a glucocorticoid receptive disease. "Glucocorticoid receptive disease" in the present context is defined as a disease which responds to glucocorticoid therapy. Diseases which are known to respond to glucocorticoid therapy are as listed below. A person skilled in the art considering the present combination therapy would consider that therapy as soon as confronted with a disease state for which he or she would have considered a treatment with a glucocorticoid.

Both inflammatory diseases as well as certain cancer types are well known to be glucocorticoid-receptive diseases.

For both types of diseases standard glucocorticoid therapies exist. It is the purpose of the present invention to combine anti-MIF therapy with these standard glucocorticoid therapies. It is possible in some instances to also reduce the standard glucocorticoid dosage, if necessary due to side effects.

In a preferred embodiment, the glucocorticoid receptive disease is a disease where a significant improvement of the sedimentation values in the erythrocyte sedimentation assay is observed. A significant improvement is an improvement as considered significant by a person skilled in the art.

Particularly preferred diseases which could be treated with the present combination therapy would thus be: Inflammatory diseases, even more preferred, the inflammatory diseases would be those diseases which act via the adaptive immune system (i.e. via B- and T-cells).

Even more preferred, the glucocorticoid receptive disease would be an allergic disease, even more preferred an autoimmune disorder.

All these diseases are known to be responsive to glucocorticoid therapy.

Particularly preferred diseases in the context of the present invention are:
- nephritis, in particular glomerulonephritis, rapidly progressive glomerulonephritis (type I, II, III or IV), systemic vasculitides (e.g. polyarteritis nodosa, Wegener's granulomatosis, Henoch-Schönlein purpura, acute proliferative glomerulonephritis)
- Lupus nephritis, ANCA nephritis, anti-GBM nephritis (e.g. Goodpasture disease), IgA nephrophaty, IgM nephropathy,
- Lupus erythematosus
- T-cell mediated immune responses, e.g. Cutaneous inflammation, like psoriasis or contact hypersensitivity
- IBD, like Ulcerative colitis and MorbusCrohn
- Multiple sclerosis
- Rheumatoid arthritis
- Uveitis
- Type 2 diabetes mellitus
- Bronchial asthma
- Contact dermatitis
- Psoriasis
- Atopic dermatitis
- Pancreatitis
- Pemphigus
- Sjögren's disease
- Behcet disease
- Horton's disease
- Scleroderma
- polymyositis

The synergistic effect has been shown for glucocorticoid receptive diseases in the examples below. The selected models are particularly suitable as the effect is shown for an antigen-antibody reaction which would be mechanistically the same for all diseases listed above.

Possible dosage forms which are envisaged by the present application are tablets, capsules, sachets or pills. The granules can be used as such as a preferred dosage form, can be filled into capsules or sachets or can be further compressed into tablets or pills.

Further dosage forms which are also encompassed by the present application are drinks or syrups, elixirs, tinctures, suspensions, solutions, hydrogels, films, lozenges, chewing gums, orally disintegrating tablets, mouthwashes, toothpaste, lip balms, medicated shampoos, nanosphere suspensions and microsphere tablets, as well as aerosols, inhalers, nebulisers, smoking or freebase powder forms and dosage forms for topical application like creams, gels, liniments or balms, lotions, ointments, ear drops, eye drops and skin patches.

Further encompassed are suppositories which can be used e.g. rectally or vaginally. All these dosage forms are well-known to a person skilled in the art.

Preferred dosage forms according to the present invention are oral forms like granules, coated granules, tablets, enteric coated tablets, pellets, suppositories and emulsions. Even more preferred are granules and tablets. Other preferred dosage forms are parenteral or topical dosage forms. A particular preferred route of administration for the anti MIF antibody is a subcutaneous or intravenous application. A preferred route of administration for the glucocorticoid is oral application (e.g. a granule, liquid, sachet or tablet). A further preferred application form for the glucocorticoid is topical application, wherein a topical application can encompass an application to the skin and/or a spray, like a nasal spray or inhaler. A further preferred route of administration for a glucocorticoid is an intravenous application.

Several glucocorticoids which are marketed as anti-inflammatory are often topical formulations, such as nasal sprays for rhinitis or inhalers for asthma. These preparations have the advantage of only affecting the targeted area, thereby reducing the side effects or potential interactions. Typical compounds are Beclometasone, Budesonide, Fluticasone, Flunisolide, Clobetasone, Mometasone and Ciclesonide. For asthma, glucocorticoids are administered as inhalants with a metered dose or dry powder inhaler. Thus, such formulations are also preferred embodiments of the present invention.

The administration can be by all known routes.

The term "combination" or "combination therapy" are used interchangeably here. They refer to a dosing regimen where the anti-MIF antibody is administered together with or sequentially to the glucocorticoid or *vice versa.* The dosing regime would be typically daily for glucocorticoids and every 2 weeks for the anti-MIF antibody. Preferred dosing regimens are:
As explained above, it is possible to administer the anti-MIF antibody together with the glucocorticoid or sequentially. "Together with" in this context means that not more than 10 minutes are between the administration of the anti-MIF antibody and the administration of the glucocorticoid. "Sequentially" means that more than 10 minutes have passed between the administration of the anti-MIF antibody and the administration of the glucocorticoid. The time period can then be more than 10 min., more than 30 minutes, more than 1 hour, more than 3 hours, more than 6 hours or more than 12 hours.

Anti-MIF antibody and steroid are principally dosed in a way to ensure that both compounds are present within the body during the same time period (for a certain time span). An anti-MIF antibody has a half life of typically 2 - 4 weeks, steroids a half life of 6-24 hours,

Therefore, the above combination therapy also explicitly encompasses a sequential dosing regimen where the skilled person takes into account the well-known half-life of the respective glucocorticoid in question and the antibody in question. In view of the fact that antibodies generally have a half-life of 2 - 4 weeks, administration of the antibody in question could be only every 2 weeks, every 3 weeks or once a month. The glucocorticoid to be administered in the inventive combination therapy with such an antibody has in a typical embodiment a half-life of 6 - 24 h; therefore, administration of the glucocorticoid could be every 5 hours, every 6 hours, three times a day, twice a day or once daily in a typical embodiment.

**Table 1: dosage dexamethasone - comparison rat/mouse/human**

| Steroid | dose in rats mg/kg/day | corresponding dose in mice mg/kg/day | corresponding human dose mg/kg/day (mg/adult/day) |
|---|---|---|---|
| Dexamethasone | 0.025 | 0.05 | 0.0041 (0.25-0.5) |
| | 0.075 | 0.15 | 0.012 (0.7-1.5) |
| | 0.25 | 0.5 | 0.041 (2.5-5.0) |

"The rule of Five", which is well known to a person skilled in the art of steroids, can be used to calculate dosing schedules between different glucocorticoids, as follows: 1 mg Dexamethasone equals approximately 5 mg Prednisolone, which in turn equals approximately 25 mg Cortisone.

The above mentioned "high dose" would be typically given in a case where an acute disorder needs to be treated as effectively as possible. It is typically not given for a longer lasting dosing schedule.

Dosing of glucocorticoids, as well as the combined dosing with antibodies, according to the present invention, however, will need to be determined by the practitioner on a case-by-case basis according to the specific disorder to be treated and the particulars of the afflicted subject.

Just as an example, which shall by no means be construed as limiting the present invention, there would be typically two options for the treatment of nephritis:
1) 500 mg Metylprednisolone/day for 3 days (high dose); followed by orally administered Prednisolone max. 30 mg/day (low dose), OR
2) orally administered Prednisolone, max. 60 mg/day (medium dose), which is then reduced over time (which can take up to 6 months).

Again, the invention is directed to the person skilled in the art who will determine the necessary dosage of both glucocorticoid and anti-MIF antibody based on his or her knowledge, e.g. based on the respective treatment guidelines, e.g. the NIH guidelines.

In a particularly preferred embodiment, the active ingredient would be an ingredient which should be delivered with a controlled, e.g. a delayed release. That is, the orally administratable dosage forms of the present invention comprising such an active ingredient might be provided with a coating. Thus, in a preferred embodiment the present invention is directed to granules with coatings and in particular to granules comprising active ingredients which shall be released in a controlled manner, whereby these granules have a coating.

More preferred, this coating is pharmacologically acceptable coating and particularly preferred is an enteric coating, a prolonged release coating or a delayed release coating; all such coatings are well known to a person skilled in the art.

A subset of *in vivo* protective anti-MIF mAbs (e.g. RAB9, RAB4, RAB0, RAM9, RAM4, RAM0), which are directed against the pro-inflammatory cytokine MIF (Macrophage Migration Inhibitory Factor) do not bind to unmodified MIF in its reduced state. By contrast, these mAbs were shown to be highly selective for a redox dependent MIF isoform.

A particularly preferred antibody is antibody RAB9.

Another particularly preferred antibody is antibody RAB4.

Yet another particularly preferred antibody is antibody RAB0.

A particularly preferred antibody is antibody RAM9.

Another particularly preferred antibody is antibody RAM4.

Yet another particularly preferred antibody is antibody RAM0.

As is shown by the present invention, the combination therapy proposed here is advantageous in that it results in the very surprising synergistic effect of both components. In view of the enormous side effects encountered by treatment with glucocorticoids, the present invention is highly suitable to provide an alternative treatment which will improve the situation of the patient under treatment. Simultaneously, the reduced side effects will also improve patient compliance which is of great importance in particular in chronic diseases. Finally, the present invention allows the practitioner to increase the results as obtained with high glucocorticoid doses even further, which can be immediately life saving in particular disease states and under particular circumstances.

The present invention will be in the following described by way of the examples, whereby the examples shall be considered by no means as limiting the present invention.

### REFERENCE EXAMPLES

### A) GCO-assay for antibody screening:

A THP1 suspension culture is centrifuged and cells are resuspended in fresh full medium to a cell density of 10⁶ cells per ml. This culture is transferred into wells of a 96-wellmicroplate (90 µl/well) and a potential anti-MIF antibody is added to give a final concentration of 75 µg/ml. Each antibody is tested in triplicate. After o/n incubation at 37°C Dexamethasone is added to give a concentration of 2 nM and after one hour incubation at 37°C LPS is added (3 ng/ml final concentration). After further six hours incubation at 37°C the supernatant is harvested and the IL-6 concentrations are determined in a commercially available ELISA. The results of the triplicates are averaged and the percentage of IL-6 secretion is determined in comparison to the control antibodies. Antibodies that result in an IL-6 secretion of less than 75% are evaluated as positive.

### B) Assay for determination of IC₅₀values

The experimental procedure is carried out as described for the screening assay with the exception that increasing amounts of antibody are used (typically from 1 - 125 nM). The resultant dose response curve is expressed as % inhibition in comparison to a negative control antibody. This curve is used for calculation of the maximum inhibitory effect of the antibody (%lnh max) and the antibody concentration that shows 50% of the maximum inhibitory effect (IC₅₀).

### C) Inhibition of cell proliferation

Serum stimulates secretion of MIF in quiescent NIH/3T3 and MIF in turn stimulates cell proliferation. Antibodies inhibiting this endogenous MIF, therefore, decrease the proliferation of quiescent NIH/3T3 cells. The reduction of proliferation is determined by the incorporation of ³H-thymidine.

1000 NIH/3T3 cells per well are incubated in a 96 well plate over the weekend at 37°C in medium containing 10% serum. Cells are then starved over night at 37°C by incubation in medium containing 0.5% serum. The 0.5% medium is removed and replaced by fresh medium containing 10% serum, 75 µg/ml antibody and 5µ Ci/ml of 3H-thymidine. After 16 hours incubation in a CO₂ incubator at 37°C cells are washed twice with 150 µl of cold PBS per well. Using a multi-channel pipette 150 µl of a 5% (w/v) TCA solution per well are added and incubated for 30 minutes at 4°C. Plates are washed with 150 µl PBS. Per well 75 µl of a 0.5M NaOH solution with 0.5% SDS are added, mixed and stored at room temperature. Samples are measured in a β-counter by mixing 5 ml of Ultima Gold (Packard) and 75 µl sample solution. Each determination is done in triplicate and the values are compared with the values of the control antibody by a t-test. Antibodies that significantly reduce proliferation (P<0.05) are evaluated as positive.

### D) Binding studies: Epitope determination of anti-MIF antibodies

Each peptide is diluted in coupling buffer to give a peptide concentration of typically 1 µg/ml added to microplates (NUNC Immobilizer™ Amino Plate F96 Clear) and incubated over night at 4°C (100 µl/well). As controls recombinant full length MIF and PBS are used. The plate is washed 3 times with 200 µl PBST and antibodies (2-4 µg/ml in PBS) are added (100 µl/well) and incubated for 2 hours at room temperature with gentle shaking. The plate is washed 3 times with 200 µl PBST and detection antibody (e.g. Fc specific anti-human IgG/HRP labeled, Sigma) is added (100 µl/well). After incubation for 1 hour at room temperature with gentle shaking, the plate is washed 3 times with 200 µl PBST. Each well is incubated with 100 µl TMB (3,3',5,5'-tetramethylbenzidine) solution (T-0440, Sigma) for 30 minutes in the dark. Staining reaction is stopped by adding 100 µl of 1.8 M H₂SO₄-solution per well. Samples are measured at 450nm.

### E) Affinity determination of Fab fragments of anti-MIF antibodies by Biacore

Typically, 40 RU units of human recombinant MIF are immobilized on a sensor chip with a CM5 (= carboxymethylated dextran) matrix (Biacore). Fab fragments are injected at a concentration range of typically 6 - 100 nM diluted in HBS-EP. After each cycle the chip is regenerated with 50 mM NaOH + 1 M NaCl. Affinities are calculated according to the 1:1 Langmuir model.

### EXAMPLES

### Introduction:

Therapy of diseases using glucocorticoid drugs is often hampered by severe side effects or a required increase of dosage. In this invention we describe that the effectiveness of glucocorticoid compounds (e.g. Dexamethasone, Hydrocortisone or Prednisolone) can be increased by combination with an anti-oxMIF antibody. Such a combination has the potential to result in an improved therapy of such disorders in comparison to the respective monotherapy and even to prolong the life expectancy of patients significantly.

In particular, with the present invention the following is achieved:
- lower doses of glucocorticoids can be given to achieve the same effect with a formerly higher dosage, resulting in less side effects
- higher efficacy will be encountered at an identical glucocorticoid dosage
- high glucocorticoid dosages can be given if desired and will have an enhanced therapeutic effect

### NEPHRITIS

### Example 1: Animal model for Crescentic Glomerulonephritis:

This model is recognized in the art as predictable to show the effects of the invention. It is suitable to show the effects of the combination therapy over the complete range of diseases which can be subsumed under the above definition of "MIF related diseases" particularly, this model is suitable for the following diseases: Lupus nephritis, anti-GBM nephritis, ANCA nephritis, IgA nephropathy. Dexamethasone is recognized in the art as predictive for a respective glucocorticoid in the treatment of a human. E.g. a preferred glucocorticoid for the treatment of lupus nephritis in a human would be Methylprednisolone,

The effect of anti-MIF monoclonal antibody (mAb) and its combination with Dexamethasone is examined in immune mediated-nephrotoxic nephritis (NTN) in Wistar Kyoto (WKY) rats. This model has a rapid onset of disease with macrophage infiltration, fibrin deposition and tissue destruction 12. NTN is induced in male WKY rats by single intravenous injection of 0.1 ml rabbit anti-rat glomerular basement membrane serum (day 0). mAb and Dexamethasone is injected intraperitoneally at time points and dosages described below. 24 hour urine samples are collected using metabolic cages before induction of glomerulonephritis and at various time intervals after induction of nephritis for quantification of proteinuria. Sections of renal tissues are frozen for subsequent analysis by immunohistology. Morphology of renal injury is assessed in haematoxylin/eosin (H&E) and periodic acid-Schiff (PAS) stained renal tissue sections. Macrophage is detected by immunoperoxidase technique and using the monoclonal mouse antibody ED1 (Serotec, UC). The animal model was described in detail (Tam FWK, Smith J, Morel D, Karkar AM, Thompson EM, Cook HT, Pusey CD: Development of scarring and renal failure in a rat model of crescentic glomerulonephritis. Nephrol Dial Transplant 14:1658-1666, 1999).

### Dosing of anti-MIF antibody

Dose range of anti-MIF antibody for human therapy:
0.2 - 25 mg/kg

Preferred dose range for human therapy: 1-10 mg/kg

The anti-MIF antibodies are 10x more potent in inhibiting human MIF than mouse or rat MIF based on *in vitro* assays (chemokinesis assay) and affinity measurements. Therefore, the dose range of the Nephritis experiment (Experiment 1c) of 2-120 mg/kg in the rat corresponds to 0.2 - 12 mg/kg in the human setting.

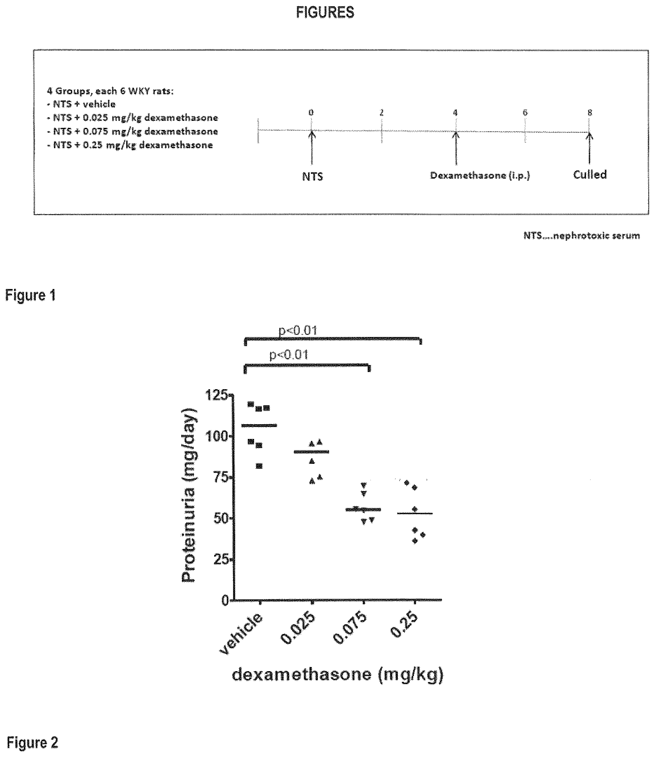

The enhanced therapeutic effect by combining a MIF antagonist with steroids is demonstrated in a rat model for Crescentic Glomerulonephritis. Different concentrations of Dexamethasone are used in a preliminary experiment to find a suboptimal steroid dose (Example 1a; a schematic diagram of the experiment is depicted in Figure 1; results are shown in Figures 2-4). In a second preliminary experiment the effectiveness of a MIF antagonist (anti-MIF antibody RAM9) is confirmed (Example 1b). Finally, a suboptimal dose of Dexamethasone is combined with said antibody (Example 1c) and this combination results in a markedly improved reduction of disease parameters. The beneficial effect seen in this combination therapy corresponds to the effect that is achieved by more than 10-fold higher steroid dose.

### Example 1a:

### Summary(see also Fig. 1)

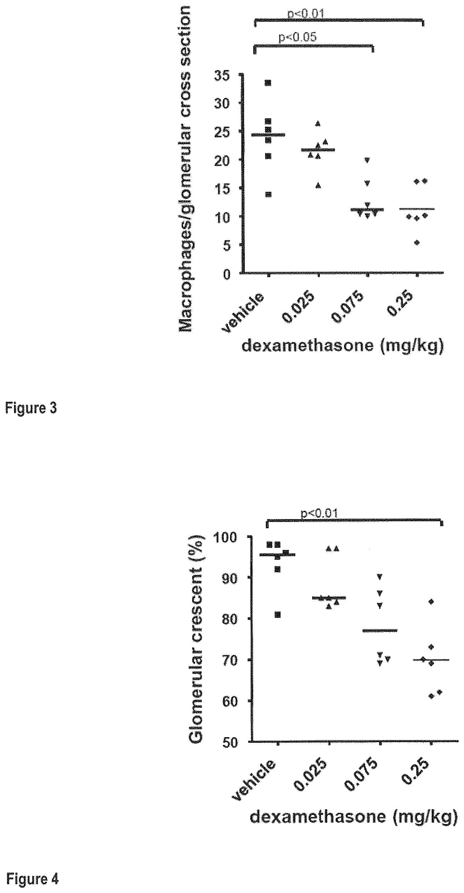

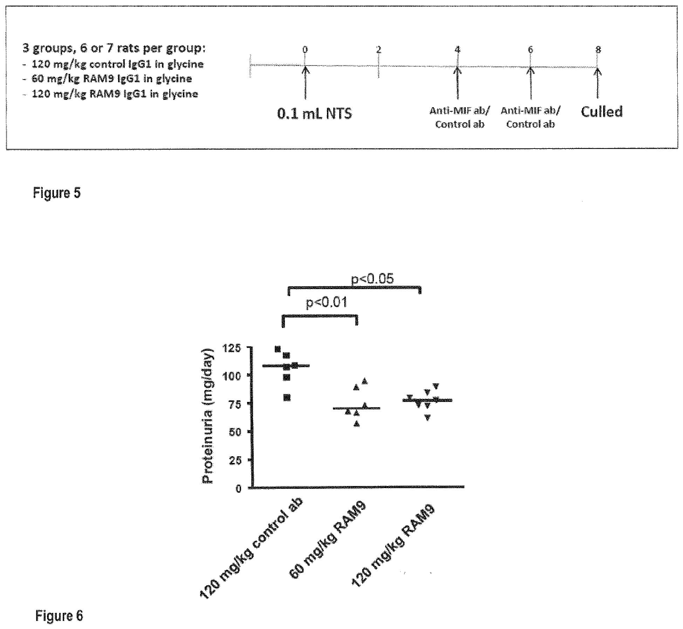

- Treatment is given 4 days after induction of glomerulonephritis in WKY rats
- Severity of renal injury is assessed after collection of urine over night (day 7 to day 8) and after culling the animals on day 8 (see Fig. 1)
- Single intraperitoneal injection of 0.25 mg/kg Dexamethasone reduces proteinuria by approximately 50% (Fig. 2), glomerular macrophage infiltration by approximately 53%)(Fig 3) and crescent counts by approximately 25% (Fig. 5).
   → 0.025 mg/kg is determined as suboptimal dexamethasone concentration
   → 0.025 mg/kg is considered as appropriate for studying a potential effect of antibody RAM9

### Example 1b:

### Summary(see also Fig. 4)

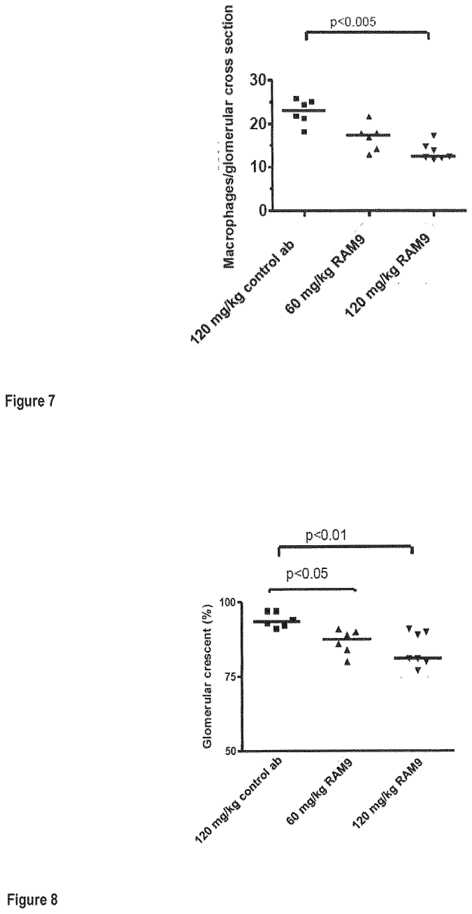

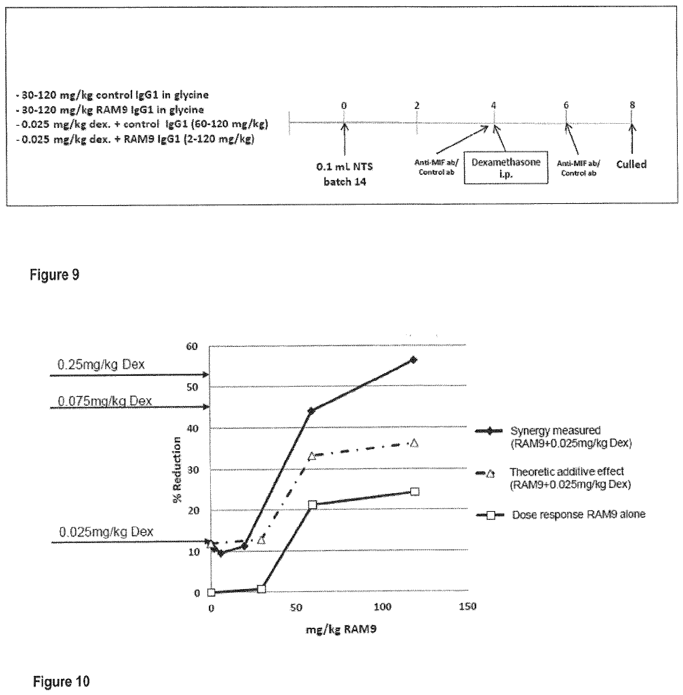

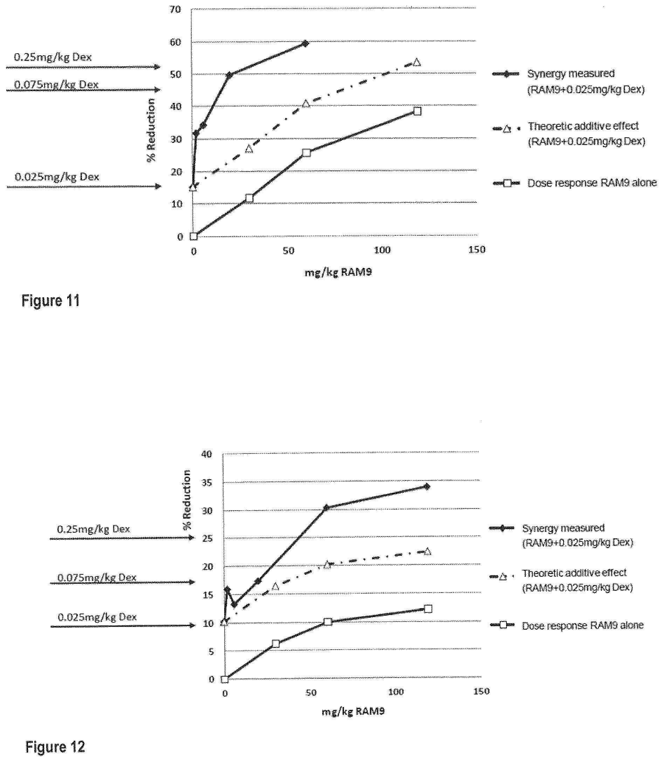

- Treatment is given 4 and 6 days after induction of glomerulonephritis in WKY rats
- Severity of renal injury is assessed after collection of urine over night (day 7 to day 8) and after culling the animals on day 8 (Fig. 5)
- Intraperitoneal injection of antibody RAM9 reduces
   - Albuminuria (FIG 6 and FIG 10): 60 mg/kg: approx. 22%, 120 mg/kg: approx. 25%
   - Macrophage infiltration (Fig. 7 and Fig 11): 60 mg/kg: approximately 26%, 120 mg/kg: approximately 38%
   - Crescent counts (Fig. 8 and Fig 12): 60 mg/kg: approximately 10%, 120 mg/kg: approximately 12%
- Furthermore, MIF, TNFα, and II-1β are reduced in urine.

### Example 1c:

### Summary (see Figure 9)

- Treatment is given 4 days (Dexamethasone + antibody) and 6 days (antibody alone) after induction of glomerulonephritis in WKY rats
- Severity of renal injury is assessed after collection of urine over night (day 7 to day 8) and after culling the animals on day 8 (Fig 9)
- Combination of antibody RAM9 and Dexamethasone shows strong synergistic effects, see Fig. 10 - 12:
   - Effect of Dexamethasone is apparently enhanced by > 10-fold

The described animal model exemplifies the beneficial effect of a combination therapy of MIF antagonists and steroids. Steroid treatment has a broad range of therapeutic applications, but shows widespread limitations (side effects at high dosages, steroid resistance, etc.). A combination therapy of steroids and MIF antibodies has the potential to open a broad new treatment opportunity in patients with e.g. inflammatory diseases, infectious diseases or cancer.

### Example 2

The following model uses NZB/NZW F1 mice that are recognized in the art an experimental model for systemic Lupus erythematosus and Lupus nephritis. Treatment of these mice is recognized as being predictable for treatment of Lupus nephritis and systemic Lupus erythematosus.

The enhanced therapeutic effect by combining a MIF antagonist with steroids is demonstrated in a mouse model for Lupus Nephritis. Different concentrations of Dexamethasone are used in a preliminary experiment to find a suboptimal steroid dose (Experiment 2a). Finally, a suboptimal dose of Dexamethasone is combined with said antibody (Experiment 2b) and this combination results in a markedly improved reduction of disease parameters.

For these experiments adult NZB/NZW F1 hybrid mice are used. These mice develop an immunologic disorder that closely resembles systemic Lupus erythematosus (SLE) in man. In the female mice the disease manifests itself by about 32-38 weeks of age with persistent proteinuria and circulating antibodies against nucleic acids.

### Example 2a:

"Dose finding of Dexamethasone in NZB/NZW F1 hybrid mice"
The goal is to determine a suboptimal dose of Dexamethasone for further synergy studies between steroid therapy and anti MIF antibodies.

The establishment of the disease in the NZB/NZW F1 mice is monitored and confirmed by measuring proteinuria. When mice show proteinuria, animals are treated i.p. with different doses of Dexamethasone or vehicle. At the end of the experiment animals are sacrificed and read out parameters are proteinuria, number of glomerular macrophages and renal histology.
Group 1: Vehicle
Group 2: Dexamethasone 0.5 mg/kg
Group 3: Dexamethasone 1.0 mg/kg
Group 4: Dexamethasone 2.0 mg/kg
Group 5: Histology control

### Example 2b:

"Synergy between anti MIF RAM9 antibody and suboptimal dexamethasone dose in NZB/NZW F1 hybrid mice" With this experiment the synergistic effect between the drug candidate RAM9 antibody and a suboptimal dose of Dexamethasone is investigated. The establishment of the disease in the NZB/NZW F1 mice is monitored and confirmed by measuring proteinuria. When mice show proteinuria, animals are treated with steroid and antibody. At the end of the experiment animals are sacrificed and read-out parameters are proteinuria, number of glomerular macrophages and renal histology.

### "Groups of animals"

Group 1: Histology control
Group 2: Control antibody BAX C3
Group 3: Anti MIF RAM9
Group 4: Dexamethasone + control antibody BAX C3
Group 5: Dexamethasone + anti MIF RAM9

**Table 2: Overview Experiment 2**

| **Experiment 2** | **Antibody** | **Steroid** |
|---|---|---|
| Lupus nephritis | - | Dexamethasone dose titration |
| Lupus nephritis | RAM9 | Dexamethasone |

### T-cell mediated Diseases

### Example 3a

The animal model used is directed to an induction of delayed-type hypersensitivity (DTH) responses to DNFB (2,4-Dinitrofluorbenzol): C57BI/6 mice are sensitized by topical application of DNFB on the abdomen. Mice are challenged by painting DNFB solution onto the mouse ears. Severity of the inflammatory responses is judged by caliper measurements of the ear thickness at 24h after challenge. At the same time, animals are sacrificed and ears are cut off, and preserved for subsequent histological analyses.

This animal model is recognized in the art as predictive for diseases related to T-cell mediated immune responses, e.g. cutaneous inflammation, like psoriasis.

The treatment schedule is as follows:
T=0d: sensitize mice
T=5d: administer anti-inflammatory therapy and challenge mice.
T=6d: quantify ear-swelling, sacrifice mice, collect ears for further histological analyses
   The experiments are carried out with the above described model to do a Dexamethasone dose titration (similarly as explained in examples 1 and 2) and to determine the effects of a combination of RAM9 antibody and Hydrocortisone (topic application).

All experiments show a synergistic effect of the combination.

### Example 3b-d: Contact hypersensitivity

### Material and Methods

The Contact hypersensitivity (CHS) response to 2,4-dinitrofluorobenzene (DNFB) was determined using a modification of an earlier model described (see Ludwig, et al., 2010, Am J Pathol 176, 1339-1345). C57BL/6 mice were sensitized by treating the shaved mouse back skin with 75 µl of DNFB (Sigma, St. Louis, MO) solution (0.5% in acetone/olive oil, 4/1). On day 5, mice were treated with topical application of hydrocortisone (on challenged ears) or vehicle (on non-challenged ears) and intravenous injection of anti-MIF antibody RAM9 or isotype control antibody. 30 min later the right ears of the mice were challenged with 20 µl 0.3% DNFB to cause a local inflammatory response. The vehicle (acetone/olive) oil was applied to the left ears as a control. The swelling response to DNFB, specified as the difference between the right and left ears, was measured 24h after challenge using a micrometer (Mitutoyo Co., Tokyo, Japan). 8 mice were used per group.

### Results

### Example 3b: Dose titration of hydrocortisone

In a first experiment, three different doses of hydrocortisone (0.3%, 1% and 3% in DAC base cream ointment) were applied without application of antibody. Vehicle without hydrocortisone served as negative control. 1 % hydrocortisone was found to be a concentration yielding sub-optimal anti-inflammatory effects in the contact dermatitis model, as determined by measuring ear thickness.

### Example 3c: Dose titration of anti-MIF antibody RAM9:

In a second experiment, 20 mg/kg anti-MIF antibody RAM9 was applied without hydrocortisone and an isotype control antibody was used as a negative control. Ear swelling, the primary measure in these experiments, was reduced significantly in anti-MIF-treated mice compared with mice treated with an isotype-control antibody or saline. As 20 mg/kg was a highly effective dose, 10 mg/kg RAM9 was used as a sub optimal dose for the subsequent experiment.

### Example 3d: Combined application of RAM9 (i.v.) and hydrocortisone (topical)

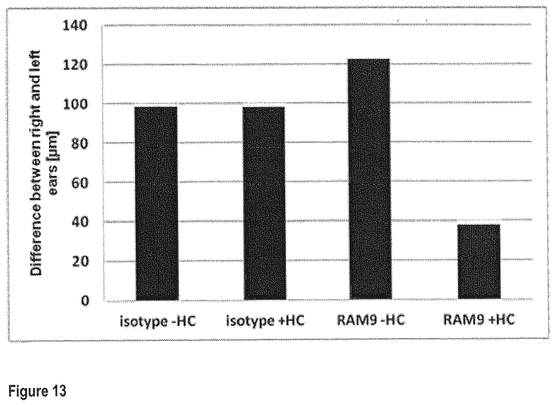

Finally, mice were treated with 10 mg/kg isotype control antibody or RAM9 alone or in combination with hydrocortisone (1% in DAC base cream). Results are shown in figure 13. Isotype control antibody treated mice showed an ear swelling of approximately 100 µm. Topical application of a suboptimal dose of hydrocortisone in combination with the isotype control antibody did not reduce ear swelling, 10 mg/kg RAM9 as monotherapy gave also a similar result as the isotype control antibody. However, thickness of the ears was markedly reduced in the group receiving both, RAM9 and hydrocortisone, and a synergistic effect of both drugs in minimization of ear swelling can be concluded.

### Conclusion

In summary, these experiments show evidence for a therapeutic synergy of suboptimal doses of RAM9 and hydrocortisone in a mouse model for contact dermatitis.

## Claims

1. An anti-MIF antibody in combination with a glucocorticoid for use in the treatment of inflammation, wherein the antibody is an anti-oxMIF antibody, and wherein the anti-MIF antibody is selected from the following group: anti-MIF antibody RAB9, i.e. an antibody with a light chain as deposited as DSM 25111 and a heavy chain as deposited as DSM 25113, RAB4, i.e. an antibody with a light chain as deposited as DSM 25110 and a heavy chain as deposited as DSM 25112 and/or RAB0, i.e. an antibody with a light chain as deposited as DSM 25114 and a heavy chain as deposited as DSM 25115, or RAM9, i.e. an antibody with a light chain as deposited as DSM 25859 and a heavy chain as deposited as DSM 25860, RAM4, i.e. an antibody with a light chain as deposited as DSM 25861 and a heavy chain as deposited as DSM 25862 or RAM0, i.e. an antibody with a light chain as deposited as DSM 25863 and a heavy chain as deposited as DSM 25864.

2. The combination for the use as claimed in claim 1 wherein the glucocorticoid is selected from the group consisting of Methylprednisolone, Prednisolone, Trimacinolone, Dexamethasone, Paramethasone, Fluorcortolone, Budesonide, Fluticasone, Flunisolide, Ciclesonide, Mometasone, Clobetasone, Cortisone and Hydrocortisone, and anti-inflammatory SEGRA's/SEGRM's.

3. The combination for the use as claimed in any of claims 1 to 2, wherein the anti-MIF antibody is selected from the group of antibody RAB9, i.e. an antibody with a light chain as deposited as DSM 25111 and a heavy chain as deposited as DSM 25113, antibody RAB4, i.e. an antibody with a light chain as deposited as DSM 25110 and a heavy chain as deposited as DSM 25112 and antibody RAB0, i.e. an antibody with a light chain as deposited as DSM 25114 and a heavy chain as deposited as DSM 25115, or RAM9, i.e. an antibody with a light chain as deposited as DSM 25859 and a heavy chain as deposited as DSM 25860, RAM4, i.e. an antibody with a light chain as deposited as DSM 25861 and a heavy chain as deposited as DSM 25862 or RAM0, i.e. an antibody with a light chain as deposited as DSM 25863 and a heavy chain as deposited as DSM 25864, preferably antibody RAM9, the glucocorticoid is selected from the group of (methyl)Prednisolone and Dexamethasone in the case of systemic administration and Budesonide in the case of local administration and the inflammation is selected from the group of nephritis, cutaneous inflammation, inflammatory bowel disease (IBD) and multiple sclerosis..

4. The combination for the use of claim 3, wherein the nephritis is selected from lupus nephritis, glomerulonephritis, IgA or IgM nephropathy, systemic vasculitides anti-GBM nephritis and rapidly progressive glomerulonephritis.

5. The combination for the use of claim 4, wherein the systemic vasculitides is selected from polyarteritis nodosa, Wegener's granulomatosis and Henoch-Schonlein purpura.

6. The combination for the use of claim 4, wherein the glomerulonephritis is selected from type I, type II, type III or type IV, and ANCA (anti-neutrophil cytoplasmic antibodies) nephritis.

7. The combination for the use as claimed in claim 4, wherein the anti-MIF antibody is RAM9, i.e. an antibody with a light chain as deposited as DSM 25859 and a heavy chain as deposited as DSM 25860, the glucocorticoid is selected from the group of (methyl)Prednisolone and Dexamethasone in the case of systemic administration and Budesonide in the case of local administration and the nephritis is Lupus nephritis.

8. The combination for the use as claimed in claim 4, wherein the anti-MIF antibody is RAM9, i.e. an antibody with a light chain as deposited as DSM 25859 and a heavy chain as deposited as DSM 25860, the glucocorticoid is selected from the group of (methyl)Prednisolone and Dexamethasone in the case of systemic administration and Budesonide in the case of local administration and the nephritis is systemic Lupus erythematosus.

9. The combination for the use as claimed in any of claims 1 to 4, wherein the anti-MIF antibody is selected from the group of antibody RAB9, i.e. an antibody with a light chain as deposited as DSM 25111 and a heavy chain as deposited as DSM 25113, antibody RAB4, i.e. an antibody with a light chain as deposited as DSM 25110 and a heavy chain as deposited as DSM 25112 and antibody RAB0, i.e. an antibody with a light chain as deposited as DSM 25114 and a heavy chain as deposited as DSM 25115, or RAM9, i.e. an antibody with a light chain as deposited as DSM 25859 and a heavy chain as deposited as DSM 25860, RAM4, i.e. an antibody with a light chain as deposited as DSM 25861 and a heavy chain as deposited as DSM 25862 or RAM0, i.e. an antibody with a light chain as deposited as DSM 25863 and a heavy chain as deposited as DSM 25864, preferably RAM9, the glucocorticoid is selected from the group of Dexamethasone, and (methyl)Prednisolone in the case of systemic administration and Budesonide or hydrocortisone in the case of local administration and the inflammation is caused by a T-cell mediated immune response.

10. The combination for the use of claim 9, wherein the T-cell mediated immune response is cutaneous inflammation.

11. The combination for the use of claim 10, wherein the cutaneous inflammation is psoriasis or contact hypersensitivity.

12. The combination for the use of any one of claims 1-11, wherein the glucocorticoid is Budesonide and wherein the Budesonide is formulated for topical application.

13. The combination for the use of claim 3, wherein the IBD is selected from ulcerative colitis and Crohn Disease.

14. A kit comprising an anti-MIF antibody in combination with a glucocorticoid, both as defined in claim 1, and instructions for use.

15. The kit of claim 14, for use in the treatment of inflammation.

## Patentansprüche

1. Anti-MIF-Antikörper in Kombination mit einem Glucocorticoid zu Verwendung bei der Behandlung von Entzündung,
wobei der Antikörper ein Anti-oxMIF-Antikörper ist, und wobei der Anti-MIF-Antikörper aus der folgenden Gruppe ausgewählt wird: Anti-MIF-Antikörper RAB9, d.h. ein Antikörper mit einer leichten Kette wie als DSM 25111 hinterlegt und einer schweren Kette wie als DSM 25113 hinterlegt, RAB4, d.h. ein Antikörper mit einer leichten Kette wie als DSM 25110 hinterlegt und einer schweren Kette wie also DSM 25112 hinterlegt und/oder RAB0, d.h. ein Antikörper mit einer leichten Kette wie als DSM 25114 hinterlegt und einer schweren Kette wie als DSM 25115 hinterlegt, oder RAM9, d.h. ein Antikörper mit einer leichten Kette wie als DSM 25859 hinterlegt und einer schweren Kette wie als DSM 25860 hinterlegt, RAM4, d.h. ein Antikörper mit einer leichten Kette wie als DSM 25861 hinterlegt und einer schweren Kette wie als DSM 25862 hinterlegt oder RAM0, d.h. ein Antikörper mit einer leichten Kette wie als DSM 25863 hinterlegt und einer schweren Kette wie als DSM 25864 hinterlegt.

2. Kombination zur Verwendung wie in Anspruch 1 beansprucht, wobei das Glucocorticoid ausgewählt wird aus der Gruppe bestehend aus Methylprednisolon, Prednisolon, Trimacinolon, Dexamethason, Paramethason, Fluorcortolon, Budesonid, Fluticason, Flunisolid, Ciclesonid, Mometason, Clobetason, Cortison und Hydrocortison, und entzündungshemmenden SEGRAs/SEGRMs.

3. Kombination zur Verwendung wie in irgendeinem der Ansprüche 1 bis 2 beansprucht, wobei der Anti-MIF-Antikörper ausgewählt wird aus der Gruppe aus Antikörper RAB9, d.h. ein Antikörper mit einer leichten Kette wie als DSM 25111 hinterlegt und einer schweren Kette wie als DSM 25113 hinterlegt, Antikörper RAB4, d.h. ein Antikörper mit einer leichten Kette wie als DSM 25110 hinterlegt und einer schweren Kette wie als DSM 25112 hinterlegt und Antikörper RAB0, d.h. ein Antikörper mit einer leichten Kette wie als DSM 25114 hinterlegt und einer schweren Kette wie als DSM 25115 hinterlegt, oder RAM9, d.h. ein Antikörper mit einer leichten Kette wie als DSM 25859 hinterlegt und einer schweren Kette wie als DSM 25860 hinterlegt, RAM4, d.h. ein Antikörper mit einer leichten Kette wie als DSM 25861 hinterlegt und einer schweren Kette wie als DSM 25862 hinterlegt oder RAM0, d.h. ein Antikörper mit einer leichten Kette wie als DSM 25863 hinterlegt und einer schweren Kette wie als DSM 25864 hinterlegt, bevorzugt Antikörper RAM9, das Glucocorticoid ausgewählt wird aus der Gruppe von (Methyl)Prednisolon und Dexamethason im Fall von systemischer Verabreichung und Budesonid im Fall von lokaler Verabreichung, und die Entzündung ausgewählt wird aus der Gruppe von Nephritis, kutaner Entzündung, chronisch-entzündlicher Darmerkrankung (IBD) und multipler Sklerose.

4. Kombination zur Verwendung gemäß Anspruch 3, wobei die Nephritis ausgewählt wird aus Lupus-Nephritis, Glomerulonephritis, IgA- oder IgM-Nephropathie, systemischen/er Vasculitiden/Vasculitis, Anti-GBM-Nephritis und Rapid-progressiver Glomerulonephritis.

5. Kombination zur Verwendung gemäß Anspruch 4, wobei die systemischen/er Vasculitiden/Vasculitis ausgewählt wird/werden aus Polyarteriitis nodosa, Wegener-Granulomatose und Purpura Schönlein-Henoch.

6. Kombination zur Verwendung gemäß Anspruch 4, wobei die Glomerulonephritis ausgewählt wird aus Typ-I-, Typ-II-, Typ-III-, Typ-IV- und ANCA (Anti-Neutrophile cytoplasmatische Antikörper)-Nephritis.

7. Kombination zur Verwendung wie in Anspruch 4 beansprucht, wobei der Anti-MIF-Antikörper RAM9 ist, d.h. ein Antikörper mit einer leichten Kette wie als DSM 25859 hinterlegt und einer schweren Kette wie als DSM 25860 hinterlegt, das Glucocorticoid ausgewählt wird aus der Gruppe von (Methyl)Prednisolon und Dexamethason im Fall von systemischer Verabreichung and Budesonid im Fall von lokaler Verabreichung, und die Nephritis Lupus-Nephritis ist.

8. Kombination zur Verwendung wie in Anspruch 4 beansprucht, wobei der Anti-MIF-Antikörper RAM9 ist, d.h. ein Antikörper mit einer leichten Kette wie als DSM 25859 hinterlegt und einer schweren Kette wie als DSM 25860 hinterlegt, das Glucocorticoid ausgewählt wird aus der Gruppe von (Methyl)Prednisolon und Dexamethason im Fall von systemischer Verabreichung und Budesonid im Fall von lokaler Verabreichung, und die Nephritis systemischer Lupus erythematodes ist.

9. Kombination zur Verwendung wie in irgendeinem der Ansprüche 1 bis 4 beansprucht, wobei der Anti-MIF-Antikörper ausgewählt wird aus der Gruppe aus Antikörper RAB9, d.h. ein Antikörper mit einer leichten Kette wie als DSM 25111 hinterlegt und einer schweren Kette wie als DSM 25113 hinterlegt, Antikörper RAB4, d.h. ein Antikörper mit einer leichten Kette wie als DSM 25110 hinterlegt und einer schweren Kette wie als DSM 25112 hinterlegt und Antikörper RAB0, d.h. ein Antikörper mit einer leichten Kette wie als DSM 25114 hinterlegt und einer schweren Kette wie als DSM 25115 hinterlegt, oder RAM9, d.h. ein Antikörper mit einer leichten Kette wie als DSM 25859 hinterlegt und einer schweren Kette wie als DSM 25860 hinterlegt, RAM4, d.h. ein Antikörper mit einer leichten Kette wie als DSM 25861 hinterlegt und einer schweren Kette wie als DSM 25862 hinterlegt oder RAM0, d.h. ein Antikörper mit einer leichten Kette wie als DSM 25863 hinterlegt und einer schweren Kette wie als DSM 25864 hinterlegt, bevorzugt RAM9, das Glucocorticoid ausgewählt wird aus der Gruppe von Dexamethason und (Methyl)Prednisolon im Fall von systemischer Verabreichung und Budesonid oder Hydrocortison im Fall von lokaler Verabreichung, und die Entzündung von einer T-Zell-vermittelter Immunantwort verursacht wird.

10. Kombination zur Verwendung gemäß Anspruch 9, wobei die T-Zell-vermittelte Immunantwort kutane Entzündung ist.

11. Kombination zur Verwendung gemäß Anspruch 10, wobei die kutane Entzündung Psoriasis oder Kontakt-Hypersensitivität ist.

12. Kombination zur Verwendung gemäß irgendeinem der Ansprüche 1-11, wobei das Glucocorticoid Budenosid ist und wobei das Budenosid für topische Anwendung formuliert ist.

13. Kombination zur Verwendung gemäß Anspruch 3, wobei die IBD ausgewählt wird aus Colitis Ulcerosa und Morbus Crohn.

14. Kit, umfassend einen Anti-MIF-Antikörper in Kombination mit einem Glucocorticoid, beide wie in Anspruch 1 definiert, und Gebrauchsanweisungen.

15. Kit gemäß Anspruch 14, zur Verwendung bei der Behandlung von Entzündung.

## Revendications

1. Anticorps anti-MIF en combinaison avec un glucocorticoïde à utiliser dans le traitement d'une inflammation, dans lequel l'anticorps est un anticorps anti-oxMIF, et dans lequel l'anticorps anti-MIF est choisi parmi le groupe suivant : anticorps anti-MIF RAB9, c'est-à-dire un anticorps avec une chaîne légère telle que déposée sous le nom DSM 25111 et une chaîne lourde telle que déposée sous le nom DSM 25113, RAB4, c'est-à-dire un anticorps avec une chaîne légère telle que déposée sous le nom DSM 25110 et une chaîne lourde telle que déposée sous le nom DSM 25112 et/ou RAB0, c'est-à-dire un anticorps avec une chaîne légère telle que déposée sous le nom DSM 25114 et une chaîne lourde telle que déposée sous le nom DSM 25115, ou RAM9, c'est-à-dire un anticorps avec une chaîne légère telle que déposée sous le nom DSM 25859 et une chaîne lourde telle que déposée sous le nom DSM 25860, RAM4, c'est-à-dire un anticorps avec une chaîne légère telle que déposée sous le nom DSM 25861 et une chaîne lourde telle que déposée sous le nom DSM 25862 ou RAM0, c'est-à-dire un anticorps avec une chaîne légère telle que déposée sous le nom DSM 25863 et une chaîne lourde telle que déposée sous le nom DSM 25864.

2. Combinaison à utiliser selon la revendication 1, dans laquelle le glucocorticoïde est choisi parmi le groupe constitué de Méthylprednisolone, Prednisolone, Trimacinolone, Dexaméthasone, Paraméthasone, Fluorcortolone, Budésonide, Fluticasone, Flunisolide, Ciclésonide, Mométasone, Clobetasone, Cortisone et Hydrocortisone, et des SEGRA/SEGRM anti-inflammatoires.

3. Combinaison à utiliser selon l'une quelconque des revendications 1 à 2, dans laquelle l'anticorps anti-MIF est choisi parmi le groupe constitué d'un anticorps RAB9, c'est-à-dire un anticorps avec une chaîne légère telle que déposée sous le nom DSM 25111 et une chaîne lourde telle que déposée sous le nom DSM 25113, d'un anticorps RAB4, c'est-à-dire un anticorps avec une chaîne légère telle que déposée sous le nom DSM 25110 et une chaîne lourde telle que déposée sous le nom DSM 25112, et d'un anticorps RAB0, c'est-à-dire un anticorps avec une chaîne légère telle que déposée sous le nom DSM 25114 et une chaîne lourde telle que déposée sous le nom DSM 25115, ou RAM9, c'est-à-dire un anticorps avec une chaîne légère telle que déposée sous le nom DSM 25859 et une chaîne lourde telle que déposée sous le nom DSM 25860, RAM4, c'est-à-dire un anticorps avec une chaîne légère telle que déposée sous le nom DSM 25861 et une chaîne lourde telle que déposée sous le nom DSM 25862 ou RAM0, c'est-à-dire un anticorps avec une chaîne légère telle que déposée sous le nom DSM 25863 et une chaîne lourde telle que déposée sous le nom DSM 25864, de préférence l'anticorps RAM9, le glucocorticoïde est choisi parmi le groupe constitué de (méthyl)Prednisolone et de Dexaméthasone dans le cas d'une administration systémique et de Budésonide dans le cas d'une administration locale et l'inflammation est choisie parmi le groupe constitué de néphrite, inflammation cutanée, maladie inflammatoire de l'intestin (IBD) et sclérose en plaques.

4. Combinaison à utiliser selon la revendication 3, dans laquelle la néphrite est choisie parmi un lupus néphrétique, une glomérulonéphrite, une néphropathie à IgA ou à IgM, une néphrite anti-GBM à vascularites systémiques et une glomérulonéphrite à progression rapide.

5. Combinaison à utiliser selon la revendication 4, dans laquelle les vascularites systémiques sont choisies parmi une polyartérite noueuse, une granulomatose de Wegener et un purpura de Henoch-Schônlein.

6. Combinaison à utiliser selon la revendication 4, dans laquelle la glomérulonéphrite est choisie parmi une néphrite de type I, de type II, de type III ou de type IV, et une néphrite ANCA (anticorps anti-cytoplasme des neutrophiles).

7. Combinaison à utiliser selon la revendication 4, dans laquelle l'anticorps anti-MIF est RAM9, c'est-à-dire un anticorps avec une chaîne légère telle que déposée sous le nom DSM 25859 et une chaîne lourde telle que déposée sous le nom DSM 25860, le glucocorticoïde est choisi parmi le groupe constitué de (méthyl)Prednisolone et de Dexaméthasone dans le cas d'une administration systémique et de Budésonide dans le cas d'une administration locale et la néphrite est un lupus néphrétique.

8. Combinaison à utiliser selon la revendication 4, dans laquelle l'anticorps anti-MIF est RAM9, c'est-à-dire un anticorps avec une chaîne légère telle que déposée sous le nom DSM 25859 et une chaîne lourde telle que déposée sous le nom DSM 25860, le glucocorticoïde est choisi parmi le groupe constitué de (méthyl)Prednisolone et de Dexaméthasone dans le cas d'une administration systémique et de Budésonide dans le cas d'une administration locale et la néphrite est un lupus érythémateux systémique.

9. Combinaison à utiliser selon l'une quelconque des revendications 1 à 4, dans laquelle l'anticorps anti-MIF est choisi parmi le groupe constitué d'un anticorps RAB9, c'est-à-dire un anticorps avec une chaîne légère telle que déposée sous le nom DSM 25111 et une chaîne lourde telle que déposée sous le nom DSM 25113, d'un anticorps RAB4, c'est-à-dire un anticorps avec une chaîne légère telle que déposée sous le nom DSM 25110 et une chaîne lourde telle que déposée sous le nom DSM 25112, et d'un anticorps RAB0, c'est-à-dire un anticorps avec une chaîne légère telle que déposée sous le nom DSM 25114 et une chaîne lourde telle que déposée sous le nom DSM 25115, ou RAM9, c'est-à-dire un anticorps avec une chaîne légère telle que déposée sous le nom DSM 25859 et une chaîne lourde telle que déposée sous le nom DSM 25860, RAM4, c'est-à-dire un anticorps avec une chaîne légère telle que déposée sous le nom DSM 25861 et une chaîne lourde telle que déposée sous le nom DSM 25862 ou RAM0, c'est-à-dire un anticorps avec une chaîne légère telle que déposée sous le nom DSM 25863 et une chaîne lourde telle que déposée sous le nom DSM 25864, de préférence RAM9, le glucocorticoïde est choisi parmi le groupe constitué de Dexaméthasone, et de (méthyl)Prednisolone dans le cas d'une administration systémique et de Budésonide ou d'hydrocortisone dans le cas d'une administration locale et l'inflammation est provoquée par une réponse immunitaire régulée par des lymphocytes T.

10. Combinaison à utiliser selon la revendication 9, dans laquelle la réponse immunitaire régulée par des lymphocytes T est une inflammation cutanée.

11. Combinaison à utiliser selon la revendication 10, dans laquelle l'inflammation cutanée est un psoriasis ou une hypersensibilité de contact.

12. Combinaison à utiliser selon l'une quelconque des revendications 1 à 11, dans laquelle le glucocorticoïde est le Budésonide et dans laquelle le Budésonide est formulé pour une application topique.

13. Combinaison à utiliser selon la revendication 3, dans laquelle la IBD est choisie parmi une colite ulcéreuse et la maladie de Crohn.

14. Kit comprenant un anticorps anti-MIF en combinaison avec un glucocorticoïde, tous deux définis dans la revendication 1, et des instructions d'utilisation.

15. Kit selon la revendication 14, destiné à être utilisé dans le traitement d'une inflammation.


### SEQUENCE LISTING

<110> Baxter Healthcare S.A.
   Baxter International Inc.
<120> COMBINATION THERAPY OF ANTI-MIF ANTIBODIES AND GLUCOCORTICOIDS
<130> 163 258
<160> 14
<170> PatentIn version 3.5
<210> 1
   <211> 214
   <212> PRT
   <213> Artificial Sequence
<220>
   <223> Light chain of RAB9
<400> 1
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Arg Ile Met Thr Tyr
               20                  25                  30
   Leu Asn Trp Tyr Gln Gln Lys Pro Gly Lys Ala Pro Lys Leu Leu Ile
           35                  40                  45
   Phe Val Ala Ser His Ser Gln Ser Gly Val Pro Ser Arg Phe Arg Gly
       50                  55                  60
   Ser Gly Ser Glu Thr Asp Phe Thr Leu Thr Ile Ser Gly Leu Gln Pro
   65                  70                  75                  80
   Glu Asp Ser Ala Thr Tyr Tyr Cys Gln Gln Ser Phe Trp Thr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 2
   <211> 214
   <212> PRT
   <213> Artificial Sequence
<220>
   <223> Light chain of RAB4
<400> 2
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Gly Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Ala Ser Gly Thr Asp Phe Thr Leu Thr Ile Ser Arg Leu Gln
   65                  70                  75                  80
   Pro Glu Asp Phe Ala Val Tyr Tyr Cys Gln Gln Tyr Gly Arg Ser Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 214
   <212> PRT
   <213> Artificial Sequence
<220>
   <223> Light chain of RAB0
<400> 3
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Gly Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Ala Ser Gly Thr Asp Phe Thr Leu Thr Ile Ser Arg Leu Gln
   65                  70                  75                  80
   Pro Glu Asp Phe Ala Val Tyr Tyr Cys Gln Gln Tyr Gly Arg Ser Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 4
   <211> 214
   <212> PRT
   <213> Artificial Sequence
<220>
   <223> Light chain of RAB2
<400> 4
   Asp Ile Gln Met Thr Gln Ser Pro Val Thr Leu Ser Leu Ser Pro Gly
   1 5 10 15
   Glu Arg Ala Thr Leu Ser Cys Arg Ala Ser Gln Ser Val Arg Ser Ser
   20 25 30
   Tyr Leu Ala Trp Tyr Gln Gln Lys Pro Gly Gln Thr Pro Arg Leu Leu
   35 40 45
   Ile Tyr Gly Ala Ser Asn Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Asn Ser Leu
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 5
   <211> 445
   <212> PRT
   <213> Artificial Sequence
<220>
   <223> Heavy chain of RAB9
<400> 5
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ser Met Asn Trp Val Arg Gln Ala Pro Gly Lys Gly Leu Glu Trp Val
           35                  40                  45
   Ser Ser Ile Gly Ser Ser Gly Gly Thr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Gly Ser Gln Trp Leu Tyr Gly Met Asp Val Trp Gly Gln Gly Thr
               100                 105                 110
   Thr Val Thr Val Ser Ser Ala Ser Thr Lys Gly Pro Ser Val Phe Pro
           115                 120                 125
   Leu Ala Pro Cys Ser Arg Ser Thr Ser Glu Ser Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Lys Thr Tyr Thr Cys Asn Val Asp His Lys Pro Ser
           195                 200                 205
   Asn Thr Lys Val Asp Lys Arg Val Glu Ser Lys Tyr Gly Pro Pro Cys
       210                 215                 220
   Pro Pro Cys Pro Ala Pro Glu Phe Leu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser Gln Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Gly Leu Pro Ser Ser Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Gln Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Arg Leu Thr Val Asp Lys Ser Arg Trp Gln
   405 410 415
   Glu Gly Asn Val Phe Ser Cys Ser Val Met His Glu Ala Leu His Asn
   420 425 430
   His Tyr Thr Gln Lys Ser Leu Ser Leu Ser Leu Gly Lys
   435 440 445
<210> 6
   <211> 454
   <212> PRT
   <213> Artificial Sequence
<220>
   <223> Heavy chain of RAB4
<400> 6
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ile Tyr
   20 25 30
   Ala Met Asp Trp Val Arg Gln Ala Pro Gly Lys Gly Leu Glu Trp Val
   35 40 45
   Ser Gly Ile Val Pro Ser Gly Gly Phe Thr Lys Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Val Asn Val Ile Ala Val Ala Gly Thr Gly Tyr Tyr Tyr Tyr
   100 105 110
   Gly Met Asp Val Trp Gly Gln Gly Thr Thr Val Thr Val Ser Ser Ala
   115 120 125
   Ser Thr Lys Gly Pro Ser Val Phe Pro Leu Ala Pro Cys Ser Arg Ser
       130                 135                 140
   Thr Ser Glu Ser Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Lys Thr Tyr
           195                 200                 205
   Thr Cys Asn Val Asp His Lys Pro Ser Asn Thr Lys Val Asp Lys Arg
       210                 215                 220
   Val Glu Ser Lys Tyr Gly Pro Pro Cys Pro Pro Cys Pro Ala Pro Glu
   225                 230                 235                 240
   Phe Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser Gln Glu Asp Pro Glu Val Gln Phe Asn Trp Tyr Val Asp Gly
           275                 280                 285
   Val Glu Val His Asn Ala Lys Thr Lys Pro Arg Glu Glu Gln Phe Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Gly Leu Pro
                   325                 330                 335
   Ser Ser Ile Glu Lys Thr Ile Ser Lys Ala Lys Gly Gln Pro Arg Glu
               340                 345                 350
   Pro Gln Val Tyr Thr Leu Pro Pro Ser Gln Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385 390 395 400
   Thr Pro Pro Val Leu Asp Ser Asp Gly Ser Phe Phe Leu Tyr Ser Arg
   405 410 415
   Leu Thr Val Asp Lys Ser Arg Trp Gln Glu Gly Asn Val Phe Ser Cys
   420 425 430
   Ser Val Met His Glu Ala Leu His Asn His Tyr Thr Gln Lys Ser Leu
   435 440 445
   Ser Leu Ser Leu Gly Lys
   450
<210> 7
   <211> 454
   <212> PRT
   <213> Artificial Sequence
<220>
   <223> Heavy chain of RAB0
<400> 7
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Trp Tyr
   20 25 30
   Ala Met Asp Trp Val Arg Gln Ala Pro Gly Lys Gly Leu Glu Trp Val
   35 40 45
   Ser Gly Ile Tyr Pro Ser Gly Gly Arg Thr Lys Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Val Asn Val Ile Ala Val Ala Gly Thr Gly Tyr Tyr Tyr Tyr
   100 105 110
   Gly Met Asp Val Trp Gly Gln Gly Thr Thr Val Thr Val Ser Ser Ala
   115 120 125
   Ser Thr Lys Gly Pro Ser Val Phe Pro Leu Ala Pro Cys Ser Arg Ser
       130                 135                 140
   Thr Ser Glu Ser Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Lys Thr Tyr
           195                 200                 205
   Thr Cys Asn Val Asp His Lys Pro Ser Asn Thr Lys Val Asp Lys Arg
       210                 215                 220
   Val Glu Ser Lys Tyr Gly Pro Pro Cys Pro Pro Cys Pro Ala Pro Glu
   225                 230                 235                 240
   Phe Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser Gln Glu Asp Pro Glu Val Gln Phe Asn Trp Tyr Val Asp Gly
           275                 280                 285
   Val Glu Val His Asn Ala Lys Thr Lys Pro Arg Glu Glu Gln Phe Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Gly Leu Pro
                   325                 330                 335
   Ser Ser Ile Glu Lys Thr Ile Ser Lys Ala Lys Gly Gln Pro Arg Glu
               340                 345                 350
   Pro Gln Val Tyr Thr Leu Pro Pro Ser Gln Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385 390 395 400
   Thr Pro Pro Val Leu Asp Ser Asp Gly Ser Phe Phe Leu Tyr Ser Arg
   405 410 415
   Leu Thr Val Asp Lys Ser Arg Trp Gln Glu Gly Asn Val Phe Ser Cys
   420 425 430
   Ser Val Met His Glu Ala Leu His Asn His Tyr Thr Gln Lys Ser Leu
   435 440 445
   Ser Leu Ser Leu Gly Lys
   450
<210> 8
   <211> 454
   <212> PRT
   <213> Artificial Sequence
<220>
   <223> Heavy chain of RAB2
<400> 8
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ile Tyr
   20 25 30
   Ala Met Asp Trp Val Arg Gln Ala Pro Gly Lys Gly Leu Glu Trp Val
   35 40 45
   Ser Gly Ile Val Pro Ser Gly Gly Phe Thr Lys Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Val Asn Val Ile Ala Val Ala Gly Thr Gly Tyr Tyr Tyr Tyr
   100 105 110
   Gly Met Asp Val Trp Gly Gln Gly Thr Thr Val Thr Val Ser Ser Ala
   115 120 125
   Ser Thr Lys Gly Pro Ser Val Phe Pro Leu Ala Pro Cys Ser Arg Ser
       130                 135                 140
   Thr Ser Glu Ser Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Lys Thr Tyr
           195                 200                 205
   Thr Cys Asn Val Asp His Lys Pro Ser Asn Thr Lys Val Asp Lys Arg
       210                 215                 220
   Val Glu Ser Lys Tyr Gly Pro Pro Cys Pro Pro Cys Pro Ala Pro Glu
   225                 230                 235                 240
   Phe Leu Gly Gly Pro Ser Val Phe Leu Phe Pro Pro Lys Pro Lys Asp
                   245                 250                 255
   Thr Leu Met Ile Ser Arg Thr Pro Glu Val Thr Cys Val Val Val Asp
               260                 265                 270
   Val Ser Gln Glu Asp Pro Glu Val Gln Phe Asn Trp Tyr Val Asp Gly
           275                 280                 285
   Val Glu Val His Asn Ala Lys Thr Lys Pro Arg Glu Glu Gln Phe Asn
       290                 295                 300
   Ser Thr Tyr Arg Val Val Ser Val Leu Thr Val Leu His Gln Asp Trp
   305                 310                 315                 320
   Leu Asn Gly Lys Glu Tyr Lys Cys Lys Val Ser Asn Lys Gly Leu Pro
                   325                 330                 335
   Ser Ser Ile Glu Lys Thr Ile Ser Lys Ala Lys Gly Gln Pro Arg Glu
               340                 345                 350
   Pro Gln Val Tyr Thr Leu Pro Pro Ser Gln Glu Glu Met Thr Lys Asn
           355                 360                 365
   Gln Val Ser Leu Thr Cys Leu Val Lys Gly Phe Tyr Pro Ser Asp Ile
       370                 375                 380
   Ala Val Glu Trp Glu Ser Asn Gly Gln Pro Glu Asn Asn Tyr Lys Thr
   385                 390                 395                 400
   Thr Pro Pro Val Leu Asp Ser Asp Gly Ser Phe Phe Leu Tyr Ser Arg
                   405                 410                 415
   Leu Thr Val Asp Lys Ser Arg Trp Gln Glu Gly Asn Val Phe Ser Cys
               420                 425                 430
   Ser Val Met His Glu Ala Leu His Asn His Tyr Thr Gln Lys Ser Leu
           435                 440                 445
   Ser Leu Ser Leu Gly Lys
       450
<210> 9
   <211> 457
   <212> PRT
   <213> Artificial Sequence
<220>
   <223> RAM0hc
<400> 9
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Trp Tyr
               20                  25                  30
   Ala Met Asp Trp Val Arg Gln Ala Pro Gly Lys Gly Leu Glu Trp Val
           35                  40                  45
   Ser Gly Ile Tyr Pro Ser Gly Gly Arg Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
           115                 120                 125
   Ser Thr Lys Gly Pro Ser Val Phe Pro Leu Ala Pro Ser Ser Lys Ser
       130                 135                 140
   Thr Ser Gly Gly Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Gln Thr Tyr
           195                 200                 205
   Ile Cys Asn Val Asn His Lys Pro Ser Asn Thr Lys Val Asp Lys Arg
       210                 215                 220
   Val Glu Pro Lys Ser Cys Asp Lys Thr His Thr Cys Pro Pro Cys Pro
   225                 230                 235                 240
   Ala Pro Glu Leu Leu Gly Gly Pro Ser Val Phe Leu Phe Pro Pro Lys
                   245                 250                 255
   Pro Lys Asp Thr Leu Met Ile Ser Arg Thr Pro Glu Val Thr Cys Val
               260                 265                 270
   Val Val Asp Val Ser His Glu Asp Pro Glu Val Lys Phe Asn Trp Tyr
           275                 280                 285
   Val Asp Gly Val Glu Val His Asn Ala Lys Thr Lys Pro Arg Glu Glu
       290                 295                 300
   Gln Tyr Asn Ser Thr Tyr Arg Val Val Ser Val Leu Thr Val Leu His
   305                 310                 315                 320
   Gln Asp Trp Leu Asn Gly Lys Glu Tyr Lys Cys Lys Val Ser Asn Lys
                   325                 330                 335
   Ala Leu Pro Ala Pro Ile Glu Lys Thr Ile Ser Lys Ala Lys Gly Gln
               340                 345                 350
   Pro Arg Glu Pro Gln Val Tyr Thr Leu Pro Pro Ser Arg Glu Glu Met
           355                 360                 365
   Thr Lys Asn Gln Val Ser Leu Thr Cys Leu Val Lys Gly Phe Tyr Pro
       370                 375                 380
   Ser Asp Ile Ala Val Glu Trp Glu Ser Asn Gly Gln Pro Glu Asn Asn
   385 390 395 400
   Tyr Lys Thr Thr Pro Pro Val Leu Asp Ser Asp Gly Ser Phe Phe Leu
   405 410 415
   Tyr Ser Lys Leu Thr Val Asp Lys Ser Arg Trp Gln Gln Gly Asn Val
   420 425 430
   Phe Ser Cys Ser Val Met His Glu Ala Leu His Asn His Tyr Thr Gln
   435 440 445
   Lys Ser Leu Ser Leu Ser Pro Gly Lys
   450 455
<210> 10
   <211> 214
   <212> PRT
   <213> Artificial Sequence
<220>
   <223> RAM0lc
<400> 10
   Asp Ile Gln Met Thr Gln Ser Pro Gly Thr Leu Ser Leu Ser Pro Gly
   1 5 10 15
   Glu Arg Ala Thr Leu Ser Cys Arg Ala Ser Gln Gly Val Ser Ser Ser
   20 25 30
   Ser Leu Ala Trp Tyr Gln Gln Lys Pro Gly Gln Ala Pro Arg Leu Leu
   35 40 45
   Ile Tyr Gly Thr Ser Ser Arg Ala Thr Gly Ile Pro Asp Arg Phe Ser
   50 55 60
   Gly Ser Ala Ser Gly Thr Asp Phe Thr Leu Thr Ile Ser Arg Leu Gln
   65 70 75 80
   Pro Glu Asp Phe Ala Val Tyr Tyr Cys Gln Gln Tyr Gly Arg Ser Leu
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 11
   <211> 448
   <212> PRT
   <213> Artificial Sequence
<220>
   <223> RAM9hc
<400> 11
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ser Met Asn Trp Val Arg Gln Ala Pro Gly Lys Gly Leu Glu Trp Val
           35                  40                  45
   Ser Ser Ile Gly Ser Ser Gly Gly Thr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Gly Ser Gln Trp Leu Tyr Gly Met Asp Val Trp Gly Gln Gly Thr
               100                 105                 110
   Thr Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 12
   <211> 214
   <212> PRT
   <213> Artificial Sequence
<220>
   <223> RAM91c
<400> 12
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Arg Ile Met Thr Tyr
               20                  25                  30
   Leu Asn Trp Tyr Gln Gln Lys Pro Gly Lys Ala Pro Lys Leu Leu Ile
           35                  40                  45
   Phe Val Ala Ser His Ser Gln Ser Gly Val Pro Ser Arg Phe Arg Gly
       50                  55                  60
   Ser Gly Ser Glu Thr Asp Phe Thr Leu Thr Ile Ser Gly Leu Gln Pro
   65                  70                  75                  80
   Glu Asp Ser Ala Thr Tyr Tyr Cys Gln Gln Ser Phe Trp Thr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 13
   <211> 457
   <212> PRT
   <213> Artificial Sequence
<220>
   <223> RAM4hc
<400> 13
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ile Tyr
               20                  25                  30
   Ala Met Asp Trp Val Arg Gln Ala Pro Gly Lys Gly Leu Glu Trp Val
           35                  40                  45
   Ser Gly Ile Val Pro Ser Gly Gly Phe Thr Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Asn Val Ile Ala Val Ala Gly Thr Gly Tyr Tyr Tyr Tyr
               100                 105                 110
   Gly Met Asp Val Trp Gly Gln Gly Thr Thr Val Thr Val Ser Ser Ala
           115                 120                 125
   Ser Thr Lys Gly Pro Ser Val Phe Pro Leu Ala Pro Ser Ser Lys Ser
       130                 135                 140
   Thr Ser Gly Gly Thr Ala Ala Leu Gly Cys Leu Val Lys Asp Tyr Phe
   145                 150                 155                 160
   Pro Glu Pro Val Thr Val Ser Trp Asn Ser Gly Ala Leu Thr Ser Gly
                   165                 170                 175
   Val His Thr Phe Pro Ala Val Leu Gln Ser Ser Gly Leu Tyr Ser Leu
               180                 185                 190
   Ser Ser Val Val Thr Val Pro Ser Ser Ser Leu Gly Thr Gln Thr Tyr
           195                 200                 205
   Ile Cys Asn Val Asn His Lys Pro Ser Asn Thr Lys Val Asp Lys Arg
       210                 215                 220
   Val Glu Pro Lys Ser Cys Asp Lys Thr His Thr Cys Pro Pro Cys Pro
   225                 230                 235                 240
   Ala Pro Glu Leu Leu Gly Gly Pro Ser Val Phe Leu Phe Pro Pro Lys
                   245                 250                 255
   Pro Lys Asp Thr Leu Met Ile Ser Arg Thr Pro Glu Val Thr Cys Val
               260                 265                 270
   Val Val Asp Val Ser His Glu Asp Pro Glu Val Lys Phe Asn Trp Tyr
           275                 280                 285
   Val Asp Gly Val Glu Val His Asn Ala Lys Thr Lys Pro Arg Glu Glu
       290                 295                 300
   Gln Tyr Asn Ser Thr Tyr Arg Val Val Ser Val Leu Thr Val Leu His
   305                 310                 315                 320
   Gln Asp Trp Leu Asn Gly Lys Glu Tyr Lys Cys Lys Val Ser Asn Lys
                   325                 330                 335
   Ala Leu Pro Ala Pro Ile Glu Lys Thr Ile Ser Lys Ala Lys Gly Gln
               340                 345                 350
   Pro Arg Glu Pro Gln Val Tyr Thr Leu Pro Pro Ser Arg Glu Glu Met
           355                 360                 365
   Thr Lys Asn Gln Val Ser Leu Thr Cys Leu Val Lys Gly Phe Tyr Pro
           370                 375                 380
   Ser Asp Ile Ala Val Glu Trp Glu Ser Asn Gly Gln Pro Glu Asn Asn
   385                 390                 395                 400
   Tyr Lys Thr Thr Pro Pro Val Leu Asp Ser Asp Gly Ser Phe Phe Leu
                   405                 410                 415
   Tyr Ser Lys Leu Thr Val Asp Lys Ser Arg Trp Gln Gln Gly Asn Val
               420                 425                 430
   Phe Ser Cys Ser Val Met His Glu Ala Leu His Asn His Tyr Thr Gln
           435                 440                 445
   Lys Ser Leu Ser Leu Ser Pro Gly Lys
       450                 455
<210> 14
   <211> 214
   <212> PRT
   <213> Artificial Sequence
<220>
   <223> RAM41c
<400> 14
   Asp Ile Gln Met Thr Gln Ser Pro Gly Thr Leu Ser Leu Ser Pro Gly
   1               5                   10                  15
   Glu Arg Ala Thr Leu Ser Cys Arg Ala Ser Gln Gly Val Ser Ser Ser
               20                  25                  30
   Ser Leu Ala Trp Tyr Gln Gln Lys Pro Gly Gln Ala Pro Arg Leu Leu
           35                  40                  45
   Ile Tyr Gly Thr Ser Ser Arg Ala Thr Gly Ile Pro Asp Arg Phe Ser
       50                  55                  60
   Gly Ser Ala Ser Gly Thr Asp Phe Thr Leu Thr Ile Ser Arg Leu Gln
   65                  70                  75                  80
   Pro Glu Asp Phe Ala Val Tyr Tyr Cys Gln Gln Tyr Gly Arg Ser Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210